Europäisches Patentamt

European Patent Office

Office européen des brevets

(19)

(11) **EP 0 950 196 B1**

(12) **FASCICULE DE BREVET EUROPEEN**

(45) Date de publication et mention
de la délivrance du brevet:
**19.03.2003   Bulletin 2003/12**

(51) Int Cl.⁷: **G01T 1/164**

(86) Numéro de dépôt international:
**PCT/FR97/02349**

(21) Numéro de dépôt: **97952103.6**

(22) Date de dépôt: **18.12.1997**

(87) Numéro de publication internationale:
**WO 98/029762 (09.07.1998 Gazette 1998/27)**

(54) **DISPOSITIF ET PROCEDE DE LOCALISATION NUCLEAIRE PAR CALCUL DE BARYCENTRE PONDERE DE DETECTEURS FONCTIONNANT EN PARALLELE, ET APPLICATION AUX GAMMA-CAMERAS**

VERFAHREN UND VORRICHTUNG ZUR LOKALISIERUNG MITTELS KERNSTRAHLUNG DURCH BERECHNUNG DES BARYZENTRUMS PARALLEL ARBEITENDER DETEKTOREN, UND VERWENDUNG IN EINER GAMMAKAMERA

DEVICE AND PROCESS FOR NUCLEAR LOCATION BY WEIGHTED BARYCENTER CALCULATION USING PARALLEL-OPERATING DETECTORS, AND APPLICATION TO GAMMA CAMERAS

(84) Etats contractants désignés:
**DE GB IT NL**

(30) Priorité:  **31.12.1996  FR 9616294**

(43) Date de publication de la demande:
**20.10.1999   Bulletin 1999/42**

(73) Titulaire: **COMMISSARIAT A L'ENERGIE ATOMIQUE**
**75015 Paris Cédex 15 (FR)**

(72) Inventeurs:
• **CHAPUIS, Alain**
**F-38950 Saint-Martin le Vinoux (FR)**
• **JANIN, Claude**
**F-38000 Grenoble (FR)**
• **NOCA, Alain**
**F-38610 Gières (FR)**

(74) Mandataire: **Audier, Philippe André et al**
**Brevalex,**
**3, rue du Docteur Lancereaux**
**75008 Paris (FR)**

(56) Documents cités:
**EP-A- 0 252 566          EP-A- 0 634 672**
**US-A- 4 611 283          US-A- 5 576 547**

**Description**

Domaine technique

La présente invention concerne un dispositif de détermination de la position d'un événement induisant un signal dans des photodétecteurs, cette position étant, par exemple, repérée par rapport à l'ensemble des photodétecteurs. Une telle position peut être repérée par le barycentre de l'événement dans un repère lié aux photodétecteurs.
L'invention s'applique en particulier à la détermination de la position d'un événement à partir de signaux fournis par des photomultiplicateurs équipant une gamma-caméra, la position étant repérée par rapport aux photomultiplicateurs eux-mêmes. On entend par gamma-caméra une caméra sensible au rayonnement gamma (γ). De telles caméras sont utilisées notamment à des fins d'imagerie médicale.

Etat de la technique antérieure

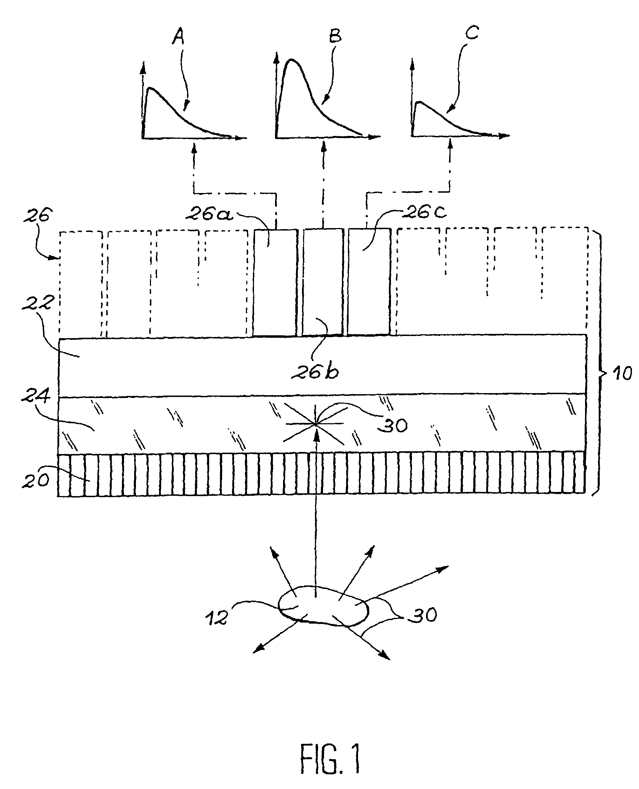

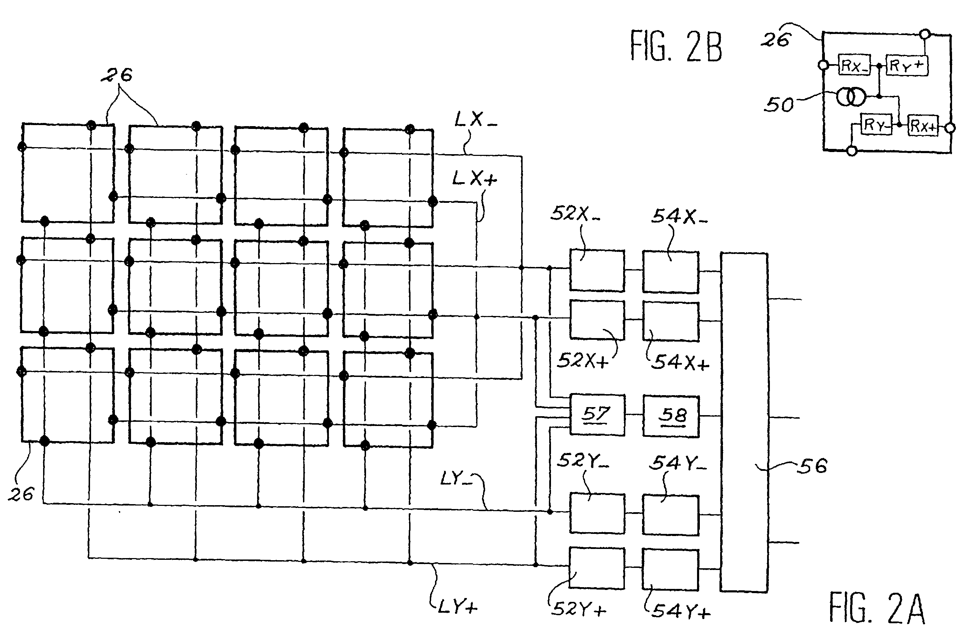

A l'heure actuelle, la plupart des gamma-caméras utilisées en médecine nucléaire sont des caméras fonctionnant selon le principe des caméras de type Anger. On peut se reporter à ce sujet au document US-3 011 057.
Les gamma-caméras permettent en particulier de visualiser la répartition, dans un organe, de molécules marquées par un isotope radioactif préalablement injecté au patient.
La structure et le fonctionnement d'une gamma-caméra connue sont exposés et résumés ci-après en référence aux figures 1, 2A et 2B annexées.
La figure 1 montre une tête de détection 10 d'une gamma-caméra disposée en face d'un organe 12 contenant des molécules marquées par un isotope radioactif.
La tête de détection 10 comporte un collimateur 20, un cristal scintillateur 22, un guide de lumière 24 et une pluralité de tubes photomultiplicateurs 26 juxtaposés de façon à couvrir une face du guide de lumière 24. Le scintillateur est, par exemple, un cristal de NaI(Tℓ).
Le collimateur 20 a pour fonction de sélectionner parmi tous les rayonnements gamma 30 émis par l'organe 12 ceux qui atteignent la tête de détection sensiblement sous incidence normale. Le caractère sélectif du collimateur permet d'augmenter la résolution et la netteté de l'image produite. Cependant, l'augmentation de la résolution se fait au détriment de la sensibilité. A titre d'exemple, pour environ 10000 photons γ émis par l'organe 12, un seul photon est effectivement détecté.
Les photons γ ayant traversé le collimateur atteignent le cristal scintillateur 22 où quasiment chaque photon γ est converti en une pluralité de photons lumineux. Dans la suite du texte on désigne par événement chaque interaction d'un photon gamma avec le cristal, provoquant une scintillation.
Les photomultiplicateurs 26 sont conçus pour émettre une impulsion électrique proportionnelle au nombre de photons lumineux reçus du scintillateur pour chaque événement.
Pour qu'un événement de scintillation puisse être localisé plus précisément, les photomultiplicateurs 26 ne sont pas directement accolés au cristal scintillateur 22 mais sont séparés de ce dernier par le guide de lumière 24.
Les photomultiplicateurs émettent un signal dont l'amplitude est proportionnelle à la quantité totale de lumière produite dans le scintillateur par un rayonnement gamma, c'est-à-dire proportionnelle à son énergie. Toutefois, le signal individuel de chaque photomultiplicateur dépend aussi de la distance qui le sépare du point d'interaction 30 du rayonnement gamma avec la matière du scintillateur. En effet, chaque photomultiplicateur délivre une impulsion de courant proportionnelle au flux lumineux qu'il a reçu. Dans l'exemple de la figure 1, des petits graphiques A, B, C montrent que des photomultiplicateurs 26a, 26b et 26c situés à différentes distances d'un point d'interaction 30 délivrent des signaux avec des amplitudes différentes.
La position du point d'interaction 30 d'un photon gamma est calculée dans la gamma-caméra à partir des signaux provenant de l'ensemble des photomultiplicateurs en effectuant une pondération barycentrique des contributions de chaque photomultiplicateur.
Le principe de la pondération barycentrique tel qu'il est mis en oeuvre dans les caméras de type Anger apparaît plus clairement en se reportant aux figures 2A et 2B annexées.
La figure 2A montre le câblage électrique d'une tête de détection 10 d'une gamma-caméra, qui relie cette caméra à une unité de formation d'une image. La tête de détection comporte une pluralité de photomultiplicateurs 26.
Comme le montre la figure 2B, chaque photomultiplicateur 26 de la tête de détection est associé à quatre résistances notées $RX^-$, $RX^+$, $RY^-$ et $RY^+$. Les valeurs de ces résistances sont propres à chaque photomultiplicateur et dépendent de la position du photomultiplicateur dans la tête de détection 10.
Les résistances $RX^-$, $RX^+$, $RY^-$ et $RY^+$ de chaque photomultiplicateur sont reliées à la sortie 50 dudit photomultiplicateur, représentée sur la figure 2B avec un symbole de générateur de courant. Elles sont d'autre part respectivement reliées à des lignes collectrices communes notées $LX^-$, $LX^+$, $LY^-$, $LY^+$, sur la figure 2A.
Les lignes $LX^-$, $LX^+$, $LY^-$ et $LY^+$ sont à leur tour reliées respectivement à des intégrateurs analogiques $52X^-$,

$52X^+$, $52Y^-$, $52Y^+$, et, par l'intermédiaire de ceux-ci à des convertisseurs analogiques/numériques $54X^-$, $54X^+$, $54Y^-$, $54Y^+$. La sortie des convertisseurs $54X^-$, $54X^+$, $54Y^-$, $54Y^+$ est dirigée vers un opérateur numérique 56. Les lignes $LX^-$, $LX^+$, $LY^-$, $LY^+$ sont par ailleurs reliées à une voie commune, dite voie énergie. Cette voie comporte également un intégrateur 57 et un convertisseur analogique/numérique 58 et sa sortie est aussi dirigée vers l'opérateur 56.

Grâce au dispositif de la figure 2, on calcule la position de l'interaction selon les équations suivantes (US-4 672 542) :

$$X = \frac{X^+ - X^-}{X^+ + X^-}$$

et

$$Y = \frac{Y^+ - Y^-}{Y^+ + Y^-}$$

dans lesquelles X et Y indiquent les coordonnées selon deux directions orthogonales de la position de l'interaction sur le cristal et dans lesquelles $X^+$, $X^-$, $Y^+$, $Y^-$ indiquent respectivement les signaux pondérés délivrés par les intégrateurs $52X^+$, $52X^-$, $52Y^+$, $52Y^-$.

Les valeurs de X et Y de même que l'énergie totale E du rayon gamma ayant interagi avec le cristal sont établies par l'opérateur numérique 56. Ces valeurs sont utilisées ensuite pour la construction d'une image comme décrit, par exemple, dans le document FR-2 669 439.

Le calcul de la position de l'interaction est entaché d'une incertitude liée aux fluctuations statistiques de Poisson du nombre de photons lumineux et du nombre de photoélectrons produits pour chaque événement, c'est-à-dire pour chaque photon gamma détecté. L'écart type de la fluctuation est d'autant plus faible que le nombre de photons ou de photoélectrons est élevé. En raison de ce phénomène, il convient de collecter la lumière le plus soigneusement possible. La résolution spatiale intrinsèque de la caméra est caractérisée par la largeur à mi-hauteur de la distribution des positions calculées pour une même source ponctuelle collimatée posée sur le cristal scintillateur.

Pour des rayons gamma d'une énergie de 140 keV, la résolution est généralement de l'ordre de 3 à 4 mm.

L'énergie d'un photon gamma détecté est calculée en faisant la somme des contributions de tous les photomultiplicateurs ayant reçu de la lumière. Elle est aussi entachée d'une fluctuation statistique. La résolution en énergie de la caméra est caractérisée par le rapport de la largeur à mi-hauteur de la distribution des énergies calculées à la valeur moyenne de la distribution, pour une même source.

La résolution en énergie est généralement de l'ordre de 9 à 11% pour des rayons gamma d'une énergie de 140 keV.

Finalement, une gamma-caméra de type Anger présente l'avantage de permettre de calculer en temps réel le barycentre des signaux des photomultiplicateurs avec des moyens très simples.

En effet, le système décrit précédemment comporte un nombre limité de composants. De plus, les résistances utilisées pour injecter le signal des photomultiplicateurs dans les lignes collectrices sont très peu coûteuses.

Des améliorations ponctuelles de la caméra de type Anger ont été proposée. Le brevet U.S-A-5 576 547 propose une méthode pour corriger le calcul de l'énergie totale reçue par les détecteurs et en déduire une position corrigée de l'événement.

Des tables de corrections sont établies en construisant pour des positions connues d'événements des histogrammes de l'énergie captée par les différents détecteurs.

Ces tables sont ensuite utilisées pour corriger la position de l'événement. La description du brevet est orientée essentiellement vers ce problème de réalisation et d'utilisation de tables. En conséquence, pratiquement rien n'est dit des claculs et transferts d'informations à partir des capteurs. En particulier, rien n'est indiqué quant à d'éventuels moyens pour augmenter la cadence de comptage des évènements.

Une caméra telle que la caméra ANGER présente cependant aussi un désavantage majeur qui est un taux de comptage réduit. On entend par taux de comptage le nombre d'événements, c'est-à-dire d'interactions entre un photon γ et le scintillateur, que la caméra est capable de traiter par unité de temps.

Une des limitations du taux de comptage provient notamment du fait que la caméra est incapable de traiter deux événements ayant lieu sensiblement simultanément en des points distincts du cristal scintillateur.

En effet, des événements simultanés mais géométriquement distincts donnent naissance à des signaux électriques qui s'empilent dans les lignes collectrices $LX^-$, $LX^+$, $LY^-$ et $LY^+$ et qui ne peuvent plus être distingués. Ces événements sont aussi "perdus" pour la formation d'une image.

Ce soucis de prise en compte et de correction des empilements est présent dans la demande de brevet européen n° 0 252 566.

Le dispositif décrit dans cette demande comporte des moyens de numérisation du signal fourni par chaque détecteur et l'énergie de chaque photodétecteur est calculée avec une correction pour tenir compte de l'énergie apportée par des événements se produisant alors que l'événement courant est encore en cours.

La limitation du taux de comptage n'est pas une contrainte trop importante dans les techniques d'imagerie médicale traditionnelles. En effet, comme indiqué ci-dessus, le collimateur arrête un très grand nombre de rayons gamma et seul un petit nombre d'événements sont effectivement détectés.

Les gamma-caméras sont cependant utilisées également dans deux autres techniques d'imagerie médicale où la limitation du taux de comptage est une contrainte rédhibitoire.

Ces techniques sont les techniques dites de "correction d'atténuation par transmission" et de "PET (Positon Emission Tomography) en coïncidence".

La technique de correction d'atténuation par transmission consiste à tenir compte, lors de la formation d'une image médicale, de l'atténuation propre du tissu du patient entourant l'organe examiné. Pour connaître cette atténuation, on mesure la transmission des rayonnements gamma vers une gamma-caméra à travers le corps du patient. A cet effet on fait prendre place au patient entre une source externe très active et la tête de détection de la gamma-caméra. Ainsi, lors de la mesure du rayonnement transmis, un nombre élevé d'événements ont lieu dans le cristal scintillateur. Le nombre élevé d'événements par unité de temps accroît aussi la probabilité d'avoir plusieurs événements sensiblement simultanés. Une caméra de type Anger classique se révèle alors inappropriée.

La technique de PET consiste à injecter au patient un élément tel que $F^{18}$ apte à émettre des positons. L'annihilation d'un positon et d'un électron libère deux photons $\gamma$ émis dans des directions opposées et ayant une énergie de 511 keV. Ce phénomène physique est mis à profit dans la technique d'imagerie PET. Dans cette technique on utilise une gamma-caméra avec au moins deux têtes de détection disposées de part et d'autre du patient. Les têtes de détection utilisées ne sont pas équipées de collimateur. En effet, un traitement électronique des informations, dit traitement de coïncidence, permet de sélectionner parmi les événements ceux qui coïncident temporellement, et de calculer ainsi la trajectoire des photons gamma.

Les têtes de détection sont donc soumises à des flux de rayonnement gamma élevé. Les gamma-caméras classiques de type Anger ont un taux de comptage généralement trop limité pour une telle application.

A titre indicatif, une gamma-caméra de type Anger peut fonctionner normalement avec une détection de $1.10^5$ événements par seconde, tandis qu'en imagerie PET il faut au moins $1.10^6$ événements par seconde pour un fonctionnement normal.

Une autre limitation des gamma-caméras du type Anger, décrites ci-dessus, tient au fait que le calcul du barycentre d'un événement est fixé définitivement par la construction de la tête de détection et notamment par le choix des résistances $RX^-$, $RX^+$, $RY^-$, $RY^+$ pour chaque photomultiplicateur. De même, le calcul de l'énergie est fixé par le câblage des photomultiplicateurs sur une voie commune (voie énergie).

Il est donc nécessaire de développer des dispositifs et des procédés permettant l'emploi de gamma-caméras à fort taux de comptage.

Exposé de l'invention

L'invention a pour objet un procédé de détermination de la position $P_0$ d'un événement par rapport à un ensemble de N photodétecteurs, cet événement induisant un signal dans les N photodétecteurs, ce procédé comportant les étapes suivantes :

a) une étape de numérisation du signal délivré par chaque photodétecteur, et de calcul d'une valeur $N_{i,j}$ représentative de l'énergie du signal délivré par chaque photodétecteur,
b) une étape de détermination, pour chaque colonne i :

- de la contribution de la colonne à l'énergie totale induite par l'événement dans l'ensemble des photodétecteurs,
- de la contribution de la colonne au barycentre, en X, de l'événement,
- de la contribution de la colonne au barycentre, en Y, de l'événement.

c) une étape de détermination :

- de l'énergie totale induite par l'événement dans l'ensemble des photodétecteurs,
- des coordonnées barycentriques $(X_0, Y_0)$ de l'événement par rapport aux N photodétecteurs.

Un tel procédé permet de traiter des données numérisées, et permet de produire un signal de position $P_0$, ou, plus précisément, un couple de coordonnées barycentriques $(X_0, Y_0)$ de l'événement par rapport à l'ensemble des N photodétecteurs.

Ce procédé permet l'exploitation de gamma-caméras à fort taux de comptage, ce qui est très avantageux dans le cas de mesures dites de "correction d'atténuation par transmission" ou de "PET en coïncidence".

Le taux de comptage élevé est atteint sans restreindre le nombre de photodétecteurs lus. Ceci est dû au parallélisme employé, et au fort "pipelinage", c'est-à-dire à la succession d'opérations simples.

Le procédé selon l'invention, tel qu'exposé ci-dessus, permet d'accélérer le calcul du barycentre des contributions numérisées des photodétecteurs, en parallélisant ce calcul.

Un procédé dans lequel les données numérisées, correspondant à chaque photodétecteur, seraient mémorisées, puis lues, ne serait pas suffisant pour que la détermination du barycentre de l'événement soit compatible avec un fort taux de comptage.

En effet, les photodétecteurs peuvent être lus en mode série ou en mode parallèle :

- mode série : les photodétecteurs sont lus l'un après l'autre sur un bus série,
- mode parallèle : plusieurs photodétecteurs d'une même ligne sont lus en même temps sur des bus colonne.

Si l'on considère qu'il faut par exemple 100 nsec (valeur non limitative donnant un ordre de grandeur) il faudra 3 $\mu$sec pour lire 30 photodétecteurs, ce qui donne un taux de comptage maximum de 330 000 coups/sec en mode série. Si, en mode parallèle, on effectue 6 pas de lecture de 100 nsec, sur 6 bus colonne, on aura lu 36 photodétecteurs en 600 nsec, ce qui correspond à un taux de comptage supérieur à 1,6 million de coups/sec. Cela impose aussi de pouvoir exploiter ce débit de façon à ce que le calcul ne soit pas pénalisant pour le taux de comptage. C'est ce que permet de réaliser le procédé selon l'invention.

Dans le cadre de l'invention, on peut évidemment remplacer les colonnes par des lignes, le principe du calcul restant le même.

Il est possible de procéder à une étape préliminaire de détection de la position présumée d'un événement. On peut alors, autour de cette position présumée, délimiter un sous-ensemble de $N_1$ photodétecteurs parmi les N photodétecteurs, seuls les signaux de ces $N_1$ photodétecteurs étant pris en compte pour réaliser les étapes b et c ci-dessus.

Selon un autre aspect, il est possible de procéder, après la détermination des coordonnées barycentriques $(X_0, Y_0)$ de l'événement par rapport aux N photodétecteurs, aux étapes

> d) détermination de la distance $d_{i,j}$ entre $P_0$ et chaque photodétecteur pris en compte dans les étapes b) et c),
> e) pondération du signal $N_{i,j}$ délivré par chaque photodétecteur pris en compte, pour obtenir une valeur de signal pondéré $N'_{i,j} = K*N_{i,j}$ où K est une fonction de $d_{i,j}$.

Il est alors possible de réaliser, ensuite :

b') une étape de détermination, à l'aide des valeurs de signal pondéré $N'_{i,j}$, pour chaque colonne i :

- de la contribution de la colonne à l'énergie totale induite par l'événement dans l'ensemble des photodétecteurs,
- de la contribution de la colonne au barycentre, en X, de l'événement,
- de la contribution de la colonne au barycentre, en Y, de l'événement.

c') Une étape de détermination, à l'aide des valeurs de signal pondéré $N'_{i,j}$ :

- de l'énergie totale induite par l'événement dans l'ensemble des photodétecteurs,
- des nouvelles coordonnées barycentriques $(X'_0, Y'_0)$ de l'événement par rapport aux N photodétecteurs.

Ainsi, on obtient une valeur de position dont la précision est améliorée.

Selon un autre aspect, il est possible de procéder, après la détermination des coordonnées barycentriques $P_0(X_0, Y_0)$ de l'événement par rapport aux N photodétecteurs, aux étapes suivantes :

> d') détermination de la distance $d_{i,j}$ entre $P_0$ et chaque photodétecteur pris en compte lors des étapes b) et c),
> f) pour chaque $d_{i,j}$, comparaison avec une valeur D,
> g) détermination, pour chaque photodétecteur, de la valeur $N_{i,j}*PD$, où PD=0 si $d_{i,j}>D$ et PD$\neq$0 si $d_{i,j}\leq D$.

On peut alors procéder à une détermination d'une nouvelle valeur de l'énergie totale en fonction des valeurs $N_{i,j}*PD$.

La précision sur l'énergie s'en trouve améliorée.

L'invention a également pour objet un dispositif pour mettre en oeuvre le procédé décrit ci-dessus.

Un tel dispositif comporte :

a) des moyens pour numériser un signal délivré par chaque photodétecteur, et pour calculer une valeur $N_{i,j}$ représentative de l'énergie du signal délivré par chaque photodétecteur,

b) des moyens pour déterminer, pour chaque colonne i :

- la contribution de la colonne à l'énergie totale induite par l'événement dans l'ensemble des photodétecteurs,
- la contribution de la colonne au barycentre, en X, de l'événement,
- la contribution de la colonne au barycentre, en Y, de l'événement.

c) des moyens pour déterminer :

- l'énergie totale induite par l'événement dans l'ensemble des photodétecteurs,
- les coordonnées barycentriques de l'événement par rapport aux N photodétecteurs.

Brève description des figures

De toute façon, les caractéristiques et avantages de l'invention apparaîtront mieux à la lumière de la description qui va suivre. Cette description porte sur les exemples de réalisation, donnés à titre explicatif et non limitatif, en se référant à des dessins annexés sur lesquels :

- la figure 1, déjà décrite, est une coupe schématique d'une tête de détection d'une caméra de type Anger connue ;
- la figure 2, déjà décrite, montre de façon schématique un dispositif de collection et de codage de signaux provenant de photomultiplicateurs de la tête de détection de la figure 1 ;
- la figure 3 représente l'interconnexion d'un ensemble de photodétecteurs,
- la figure 4 représente un dispositif de lecture d'un ensemble de photodétecteurs, pour la mise en oeuvre de l'invention,
- la figure 5 représente la structure d'un opérateur colonne pour un mode de réalisation de l'invention,
- la figure 6 représente la structure d'un système de calcul pour un mode de réalisation de l'invention,
- les figures 7A et 7B sont des exemples de fonction K,
- la figure 8 représente des étapes pour le calcul d'une contribution pondérée de chaque photodétecteur,
- la figure 9 représente la structure d'un système de calcul pour un autre mode de réalisation de l'invention,
- la figure 10 représente des étapes pour le calcul d'une contribution pondérée de chaque photodétecteur et pour la détermination des photodétecteurs à prendre en compte dans le calcul de l'énergie,
- la figure 11 représente la structure d'un système de calcul pour un autre mode de réalisation de l'invention,
- la figure 12 représente la structure d'un opérateur colonne pour un autre mode de réalisation de l'invention,
- la figure 13 représente la structure d'un système de calcul pour un autre mode de réalisation de l'invention,
- la figure 14 représente un circuit associé à un photodétecteur, pour le traitement des données de ce photodétecteur ;
- les figures 15A et 15B représentent un signal analogique fourni par un photodétecteur (figure 15A), ainsi que le signal analogique numérisé correspondant (figure 15B) ;
- la figure 16 représente un mode de réalisation d'un dispositif pour la détermination d'une position présumée d'un événement.

Description détaillée de modes de réalisation de l'invention

L'invention va être décrite de manière détaillée pour des photomultiplicateurs d'une gamma-caméra. Cependant, cette description vaut aussi pour des photodétecteurs quelconques, ne faisant pas nécessairement partie d'une gamma-caméra.

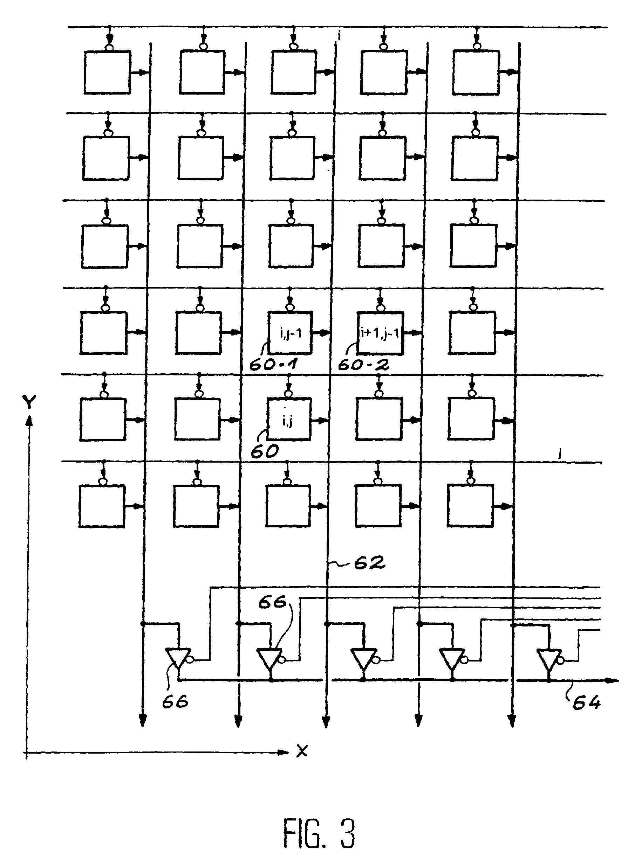

La figure 3 représente un ensemble de photomultiplicateurs 60, 60-1, 60-2,... constituant une tête de gamma-caméra. Chaque photomultiplicateur est repéré par sa position (i,j) dans l'ensemble des photomultiplicateurs. Plus précisément, on désigne par $XC_{i,j}$ et $YC_{i,j}$ les coordonnées, suivant deux axes X, Y, du centre du photomultiplicateur i, j.

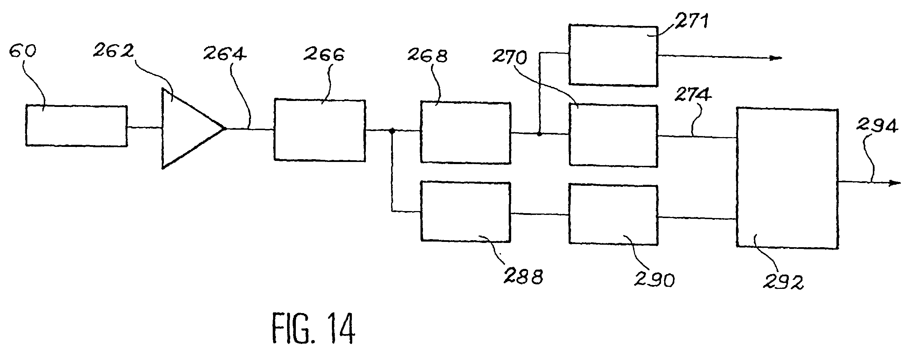

Le signal issu de chacun des photomultiplicateurs est numérisé et traité individuellement (intégration, corrections, etc.). Chaque photomultiplicateur possède un niveau de registre de stockage qui permet de mémoriser la contribution de chaque photomultiplicateur lors de la détection d'un événement. Ces aspects seront traités plus loin en liaison avec les figures 13-14B.

Le réseau de photomultiplicateurs est organisé en lignes et en colonnes, et toutes les sorties des registres de stockage des photomultiplicateurs d'une même colonne sont connectés sur un bus 62 (bus colonne). Les bus colonne peuvent être rassemblés en un bus série 64.

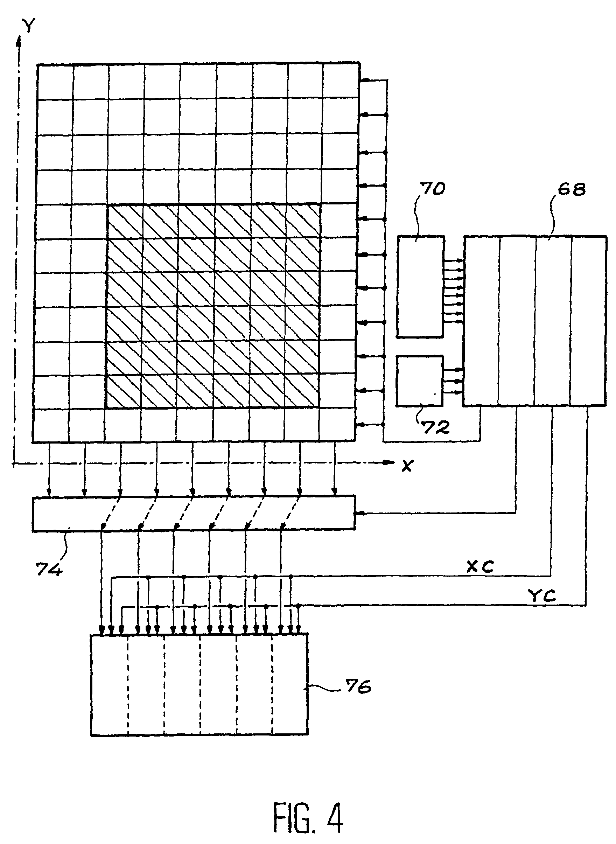

Des moyens 66 permettent de sélectionner chaque colonne indépendamment des autres. Ces moyens 66 sont par exemple commandés par un séquenceur de lecture 68 (figure 4).

Lorsque le réseau de photomultiplicateurs n'est pas trop important, on peut prendre en compte l'ensemble des photomultiplicateurs.

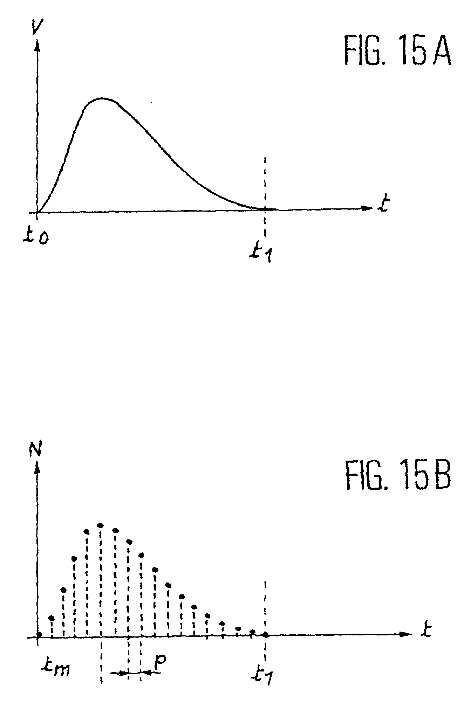

Lorsque le réseau de photomultiplicateurs est important (par exemple entre 50 et 100 photomultiplicateurs) , une position grossière de l'événement est d'abord déterminée, par exemple par une méthode qui sera décrite plus loin, en liaison avec la figure 15.

Le fait de connaître la zone d'interaction de l'événement permet déjà de limiter le nombre de photomultiplicateurs à prendre en compte pour calculer plus précisément la position et l'énergie de l'interaction. En effet, seules les deux couronnes de photomultiplicateurs qui entourent le photomultiplicateur lieu de l'interaction portent en général de l'information significative.

La précision de la position présumée étant insuffisante, on est cependant souvent appelé à lire une zone plus large que celle strictement nécessaire (par exemple souvent jusqu'à 25 ou 30 lectures par événement). La durée de lecture des registres de stockage pour traiter un événement est un passage obligé qui influe directement sur le taux de comptage de la machine (nombre d'événements traités par seconde).

La figure 4 illustre de manière plus précise un dispositif permettant de mettre en oeuvre l'invention.

Un séquenceur de lecture 68 permet de lire le contenu du registre de stockage de chaque photomultiplicateur. Soit $N_{i,j}$ le contenu du registre de stockage du photomultiplicateur de colonne i et de ligne j. $N_{i,j}$ représente en fait, par exemple, une intégrale numérique du signal délivré par le photomultiplicateur i,j en réponse à un événement.

Des moyens 70 permettent de déterminer une position présumée, ou grossière de l'événement. Ces moyens seront décrits plus loin de manière plus détaillée (figure 15).

Le séquenceur de lecture 68 commande l'adressage des colonnes par un multiplexeur 74. La contribution de chaque colonne à l'énergie totale, à la composante en X du barycentre (XC) et à la composante en Y du barycentre (YC) est transférée à un système de calcul 76, soit par l'adressage des colonnes via le multiplexeur, soit directement par le séquenceur de calcul 68.

Le nombre de colonnes et de lignes pris en compte autour de chaque position présumée est imposé par l'imprécision de la détermination. Sur la figure 4, ce sont 36 (6x6) photomultiplicateurs qui sont pris en compte. D'une manière générale, pour un champ de N photomultiplicateurs on peut être amené à prendre en compte $N_1$ photomultiplicateurs ($N_1<N$) en fonction de la précision voulue : sur la figure 4, N=11x9 et $N_1$=6x6.

Par conséquent, à chaque événement correspond une position présumée (PP) et, à chaque position présumée, on fait correspondre un ensemble de $N_1$ (36) photomultiplicateurs situés sur 6 lignes et 6 colonnes successives de façon à ce que tous les photomultiplicateurs qui ont stocké de l'information soient retenus. A chaque pas de lecture (tous les 100 nsec), le séquenceur de lecture 68 fournit, en fonction de la position présumée :

- les commandes nécessaires au multiplexeur 74 pour qu'il puisse orienter les bus colonnes à lire vers l'organe de calcul 76,
- les signaux de sélection de ligne de façon à présenter sur les bus colonne les registres de stockage commandés par la ligne n pour le premier temps de lecture, puis ceux commandés par la ligne n+1 pour le deuxième, et ceci jusqu'à ceux de la ligne n+5 pour le sixième temps de lecture,
- les coordonnées XC et YC des centres des photomultiplicateurs présentés sur les bus colonne.

Le séquenceur peut être réalisé sous forme d'EPROM. A chaque position présumée correspond une page mémoire dans laquelle on décrit les commandes et les valeurs nécessaires au calcul. Cette page est lue ligne par ligne à l'aide d'un compteur 72 qui active les adresses basses des EPROM.

Le calcul de l'énergie et/ou de la position, en X et en Y, d'un événement peut être résumé de la manière suivante.

a) l'énergie de l'événement est la somme des contributions de tous les photomultiplicateurs qui entourent la position présumée :

$$E= \sum_{i,j} N_{i,j}$$

Cette expression peut aussi s'écrire :

$$E = \sum_i \left[ \sum_j N_{i,j} \right] ,$$

où

$$\sum_j N_{i,j}$$

représente la somme des contributions des 6 photomultiplicateurs de la colonne i, l'énergie E étant la somme des énergies obtenues sur les 6 colonnes.

b) La position de l'événement est calculée par la méthode du barycentre :

- 

$$X = \sum_{i,j} \left( XC_{i,j} * N_{i,j} \right) / \sum_{i,j} N_{i,j} ,$$

où $XC_{i,j}$ est la coordonnée en X du centre du photomultiplicateur situé sur la colonne i et la ligne j,

- 

$$Y = \sum_{i,j} \left( YC_{i,j} * N_{i,j} \right) / \sum_{i,j} N_{i,j} ,$$

où $YC_{i,j}$ est la coordonnée en Y du centre du photomultiplicateur situé sur la colonne i et la ligne j,
  Comme précédemment, on peut aussi écrire :

- 

$$X = \sum_i \left[ \sum_j \left( XC_{i,j} * N_{i,j} \right) \right] / \sum_{i,j} N_{i,j} ,$$

où

$$\sum_j \left( XC_{i,j} * N_{i,j} \right)$$

est la contribution au barycentre en X des 6 photomultiplicateurs de la colonne i,

- 

$$Y = \sum_i \left[ \sum_j \left( YC_{i,j} * N_{i,j} \right) \right] / \sum_{i,j} N_{i,j} ,$$

où

$$\sum_j \left( YC_{i,j} * N_{i,j} \right)$$

est la contribution au barycentre en Y des 6 photomultiplicateurs de la colonne i,

Si on considère non plus seulement 6x6 photomultiplicateurs, mais un ensemble de $N_1$ photomultiplicateurs, les formules ci-dessus s'appliquent toujours, les sommes étant appliquées aux lignes et aux colonnes correspondantes.

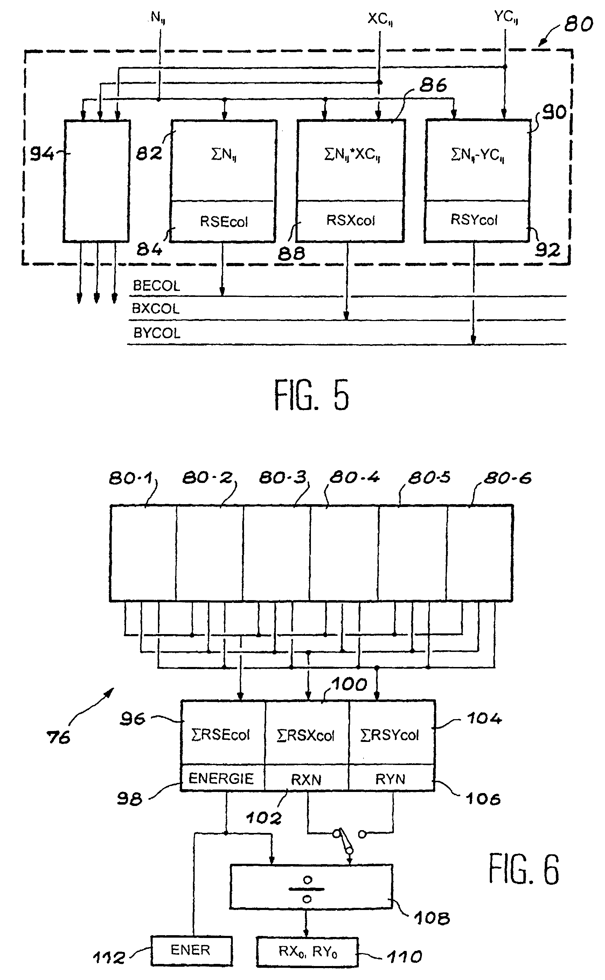

La structure du système de calcul 76 va être décrite de manière plus précise en liaison avec les figures 5 et 6.

La figure 5 représente des moyens 80 associés à chaque colonne et dénommés par la suite opérateur colonne.

Celui-ci reçoit, en plus des valeurs $N_{i,j}$ des photomultiplicateurs de la colonne, les coordonnées $XC_{i,j}$ et $YC_{i,j}$ des centres des photomultiplicateurs correspondants, par exemple fournies par le séquenceur de lecture 68. Les coordonnées en X ne sont pas obligatoirement identiques pour une même colonne car elles doivent tenir compte de la position réelle du photomultiplicateur au sein du champ de la gamma-caméra. Il en va de même pour les coordonnées en Y. Dans l'exemple donné, chaque sortie de bus colonne est connecté à l'entrée d'un opérateur colonne 80.

Chaque opérateur colonne 80 accomplit trois opérations, de préférence en parallèle.

Une première opération consiste à calculer la contribution de la colonne à l'énergie. Par exemple, après avoir été initialisé en début de séquence, un accumulateur 82 fait la somme des valeurs $N_{i,j}$ des 6 photomultiplicateurs de la colonne et stocke le résultat dans un registre 84 (RSEcol). Les sorties des 6 registres (RSEcol1 à RSEcol6) sont regroupées sur un bus commun BECOL.

Une deuxième opération consiste à calculer la contribution de la colonne au barycentre en X. Par exemple, après avoir été initialisé en début de séquence, un multiplieur-accumulateur 86 effectue la somme des contributions au barycentre en X des 6 photomultiplicateurs de la colonne, et stocke le résultat dans un registre 88 (RSXcol). Les sorties des 6 registres (RSXcol1 à RSXco16) sont regroupées sur un bus commun BXCOL.

Une troisième opération consiste à calculer la contribution de la colonne au barycentre en Y. Par exemple, après avoir été initialisé, un second multiplieur-accumulateur 90 effectue la somme des contributions au barycentre en Y des 6 photomultiplicateurs de la colonne, et stocke le résultat dans un registre 92 (RSYcol). Les sorties des 6 registres (RSYcol1 à RSYco16) sont regroupées sur un bus commun BYCOL.

A la fin des 6 temps de lecture, les 6 opérateurs colonne ayant effectué leur travail deviennent disponibles pour une nouvelle lecture puisque les résultats de la première lecture sont mémorisés.

En parallèle à ces opérations de calcul, les valeurs $N_{i,j}$ des photomultiplicateurs ainsi que les coordonnées $XC_{i,j}$ et $YC_{i,j}$ sont mémorisées dans un système 94 de type FIFO de façon à pouvoir être utilisées par la suite.

Un regroupement des contributions à l'énergie et aux coordonnées du barycentre en X et en Y est ensuite effectué. Ce regroupement va être décrit à l'aide de la figure 6 où les références 80-1, ..., 80-6 désignent 6 opérateurs colonne du type décrit ci-dessus en liaison avec la figure 5.

Un accumulateur 96, après avoir été initialisé en début de séquence, alimenté par le bus BECOL, effectue la somme des six registres RSEcol1 à RSEcol6 et stocke le résultat dans un registre 98 (ENERGIE). Le contenu de ce registre représente la somme des contributions à l'énergie des 36 photomultiplicateurs entourant la position présumée, donc l'énergie de l'événement.

Un second accumulateur 100, alimenté par le bus BXCOL, effectue la somme des six registres RSXcol1 à RSXcol6, et stocke le résultat dans un registre 102 (RXN). Le contenu de ce registre représente

$$\sum_i \left[ \sum_j (XC_{ij} * N_{ij}) \right].$$

Un troisième accumulateur 104, alimenté par le bus BYCOL, effectue la somme des six registres RSYcol1 à RSYcol6, et stocke le résultat dans un registre 106 (RYN)- Le contenu de ce registre représente

$$\sum_i \left[ \sum_j (YC_{ij} * N_{ij}) \right].$$

Les registres RSEcol, RSXcol et RSYcol sont ensuite libérés de façon à pouvoir être utilisés par les opérateurs colonne, et les accumulateurs sont ainsi de nouveau disponibles pour traiter un nouvel événement.

Enfin, les coordonnées $X_0$ et $Y_0$ du point d'interaction de l'événement sont calculées en effectuant deux divisions à l'aide d'un diviseur 108 :

$$X_0 = RXN/ENERGIE,$$

et

$$Y_0 = RYN/ENERGIE,$$

et ceci en moins de 6 temps de lecture pour pouvoir libérer les registres de stockage 98, 102, 106. Les diviseurs intégrés pipelinés du commerce (de type RAYTHEON 3211 par exemple) sont capables d'assumer largement ces performances et permettent même de n'utiliser qu'un seul boîtier, en effectuant les deux divisions successivement.

On obtient donc la position $P_0$ de l'événement dont les coordonnées $X_0$ et $Y_0$ sont stockées dans un registre 110 ($RX_0$ et $RY_0$). Parallèlement aux divisions, l'énergie est pipelinée depuis le registre 98 vers un registre 112 de façon à libérer le registre 98 pour l'événement suivant.

Les valeurs $X_0$, $Y_0$ obtenues fournissent un certain résultat, ayant une certaine précision dont on peut se satisfaire dans certaines circonstances.

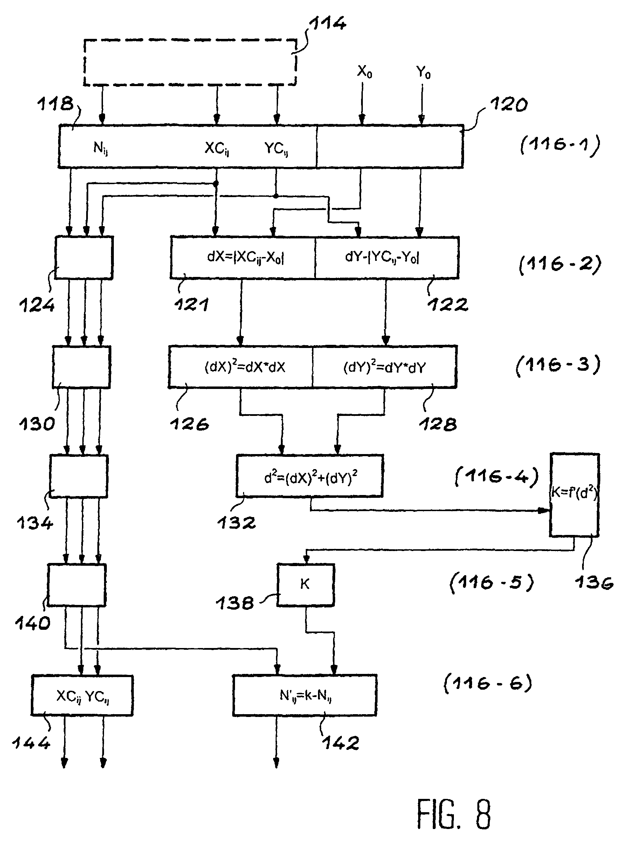

Cependant, il est apparu que l'on peut améliorer sensiblement la précision du résultat (c'est-à-dire la résolution spatiale) en adjoignant à ce calcul un calcul de barycentre dit pondéré. Nous allons montrer dans ce qui suit, en liaison avec les figures 8 à 13, comment peut être implanté ce calcul dans un système parallèle compatible avec ce qui précède.

Dans la suite, on appellera barycentre brut le résultat obtenu à l'aide du procédé déjà décrit, par opposition au barycentre pondéré.

Une fois le barycentre brut $P_0(X_0, Y_0)$ connu, on peut calculer la distance $d_{i,j}$ du centre de chaque photomultiplicateur à $P_0$ et pondérer la valeur $N_{i,j}$ en calculant un nouvel $N_{i,j}$ appelé $N'_{i,j}$, tel que $N'_{i,j} = K*N_{i,j}$, où K est une fonction de $d_{i,j}$.

Cette fonction K est déterminée de manière empirique et adaptée à chaque type de photomultiplicateur et à chaque géométrie de collection de la lumière.

La fonction K, généralement:

- est inférieure à 1, pour $d_{i,j}$ faible (pour minimiser la contribution du photomultiplicateur qui reçoit l'événement quand celui-ci est proche de son centre),
- est supérieure à 1 quand $d_{i,j}$ est de l'ordre de grandeur de la dimension du photomultiplicateur (pour renforcer la contribution des photomultiplicateurs de première couronne, c'est-à-dire des photomultiplicateurs les plus proches du photomultiplicateur qui reçoit l'événement),
- tend vers zéro, lorsque $d_{i,j}$ devient grand (pour diminuer la contribution des photomultiplicateurs au fur et à mesure qu'ils sont plus loin du lieu de l'interaction, car le rapport signal/bruit de leur contribution devient de plus en plus mauvais).

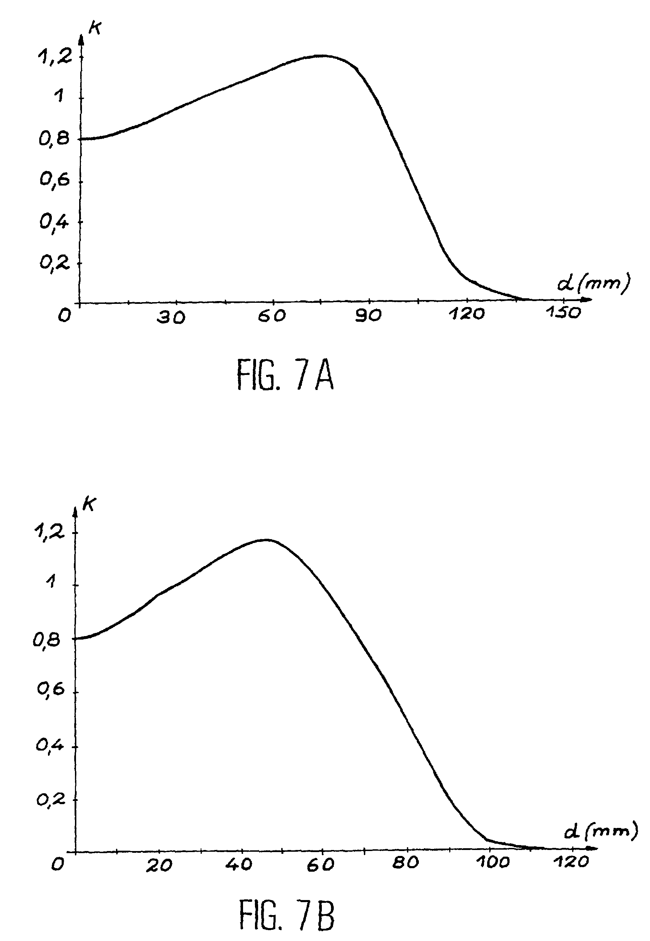

Un premier exemple de fonction K(d) est donné sur la figure 7A. Ce premier exemple correspond à des photomultiplicateurs carrés de 75 mm. Des valeurs de K, pour des valeurs particulières de d (avec un pas de 5 mm), sont données dans le tableau I ci-dessous.

TABLEAU I

| d | K | d | K |
|---|---|---|---|
| 0 | 0,8 | 80 | 1,18 |
| 5 | 0,809 | 85 | 1,13 |
| 10 | 0,826 | 90 | 1 |
| 15 | 0,85 | 95 | 0,831 |
| 20 | 0,883 | 100 | 0,663 |
| 25 | 0,916 | 105 | 0,494 |
| 30 | 0,95 | 110 | 0,325 |
| 35 | 0,983 | 115 | 0,117 |
| 40 | 1,016 | 120 | 0,1 |
| 45 | 1,05 | 125 | 0,065 |

TABLEAU I   (suite)

| d | K | d | K |
|---|---|---|---|
| 50 | 1,083 | 130 | 0,035 |
| 55 | 1,116 | 135 | 0,015 |
| 60 | 1,15 | 140 | 0 |
| 65 | 1,176 | 145 | 0 |
| 70 | 1,194 | 150 | 0 |
| 75 | 1,2 | - | - |

Un second exemple de fonction K(d) est donné sur la figure 7B. Ce second exemple correspond à des photomultiplicateurs hexagonaux de 60 mm. Des valeurs de K, pour des valeurs particulières de d (avec un pas de 5 mm), sont données dans le tableau II ci-dessous.

TABLEAU II

| d | K | d | K |
|---|---|---|---|
| 0 | 0,8 | 65 | 0,863 |
| 5 | 0,813 | 70 | 0,744 |
| 10 | 0,856 | 75 | 0,619 |
| 15 | 0,906 | 80 | 0,475 |
| 20 | 0,963 | 85 | 0,319 |
| 25 | 1,006 | 90 | 0,181 |
| 30 | 1,056 | 95 | 0,081 |
| 35 | 1,106 | 100 | 0,025 |
| 40 | 1,15 | 105 | 0,013 |
| 45 | 1,169 | 110 | 0 |
| 50 | 1,144 | 115 | 0 |
| 55 | 1,075 | 120 | 0 |
| 60 | 0,975 | - | - |

Du point de vue de la réalisation, le calcul du barycentre pondéré est réalisé de la même manière que le barycentre brut, après avoir remplacé $N_{i,j}$ par $N'_{i,j}$. On fait suivre le calcul du barycentre brut par une opération de pondération avec la fonction K, appelée fonction de pondération. Dans le cas de l'exemple déjà donné (on retient un ensemble de photomultiplicateurs définis par 6 lignes et 6 colonnes), cette tache est effectuée par 6 opérateurs, chacun d'eux étant capable de traiter 6 contributions en 6 temps de lecture.

Une réalisation simple du calcul du barycentre pondéré est donnée en figure 8.

Les valeurs de $N_{i,j}$, $XC_{i,j}$, $YC_{i,j}$, qui intervenaient dans le calcul du barycentre brut ont été mémorisées dans des mémoires FIFO désignées globalement par la référence 114 (on a déjà mentionné ci-dessus la mémoire 94 (figure 5) dans laquelle $N_{i,j}$ est stockée). Le calcul est organisé selon les étapes 116-1, ..., 116-6 suivantes, pour chaque opérateur pondération travaillant sur une colonne :

- 116-1 : récupération de la première valeur $N_{i,j}$ et des coordonnées du centre du photomultiplicateur correspondant $XC_{i,j}$ et $YC_{i,j}$ (première valeur écrite dans la FIFO, donc première valeur lue) et stockage dans un registre d'entrée 118 de l'opérateur de pondération. Parallèlement mise en mémoire 119, à l'entrée de l'opérateur, de $X_0$ et $Y_0$. Cette mémorisation n'est pas répétée pour les 5 acquisitions de $N_{i,j}$, $XC_{i,j}$ et $YC_{i,j}$ suivantes puisque $P_0$ reste le même.
- 116-2 : calcul de $dX=|XC_{i,j}-X_0|$, et parallèlement de $dY=|YC_{i,j}-Y_0|$, et mise en mémoire 120, 122, 124, de dX, dY, $N_{i,j}$, $XC_{i,j}$ et $YC_{i,j}$. Il y a ensuite libération des registres servant à l'étape 116-1, qui peuvent alors recueillir les valeurs relatives au photomultiplicateur suivant. Celui-ci est traité comme le précédent et ainsi de suite jusqu'au sixième.
- 116-3 : calcul de $(dX)^2=dX*dX$, et parallèlement de $(dY)^2=DY*DY$, et mise en mémoire 126, 128, 130 de $(dX)^2$,

$(dY)^2$, $N_{i,j}$, $XC_{i,j}$ et $YC_{i,j}$. Puis il y a libération des registres de sortie de l'étape 116-2,

- 116-4 : calcul de $d^2=(dX)^2+(dY)^2$ et stockage de $d^2$, $N_{i,j}$, $XC_{i,j}$ et $YC_{i,j}$ dans des registres 132, 134 ; puis, libération des registres de sortie de l'étape 116-3.
- 116-5 : adressage d'une EPROM 136 contenant la fonction $K=f'(d^2)$, par le registre contenant $d^2$. Ceci évite d'avoir à extraire la racine carrée de $d^2$, sachant qu'il est facile d'obtenir $K=f'(d^2)$ quand on connaît $K=f(d)$. Il y a ensuite stockage de K, $N_{i,j}$, $XC_{i,j}$ et $YC_{i,j}$ dans des registres 138, 140 et libération des registres de sortie de l'étape 116-4.
- 116-6 : calcul de $N'_{i,j}=K*N_{i,j}$ et stockage de $N'_{i,j}$, $XC_{i,j}$ et $YC_{i,j}$ dans des registres 142, 144 ; puis il y a libération des registres de sortie de l'étape 116-5.

Ainsi découpé et pipeliné le calcul de $N'_{i,j}$ est facilement réalisable car chaque pas de calcul est suffisamment simple pour être réalisé pendant la durée d'un pas de lecture (typiquement 100 nsec).

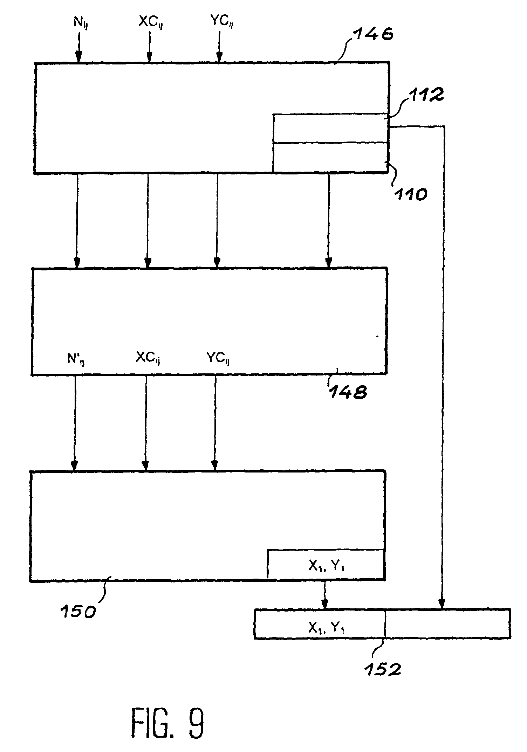

La figure 9 représente schématiquement un système de calcul selon l'invention, pour la mise en oeuvre du calcul du barycentre brut, puis du barycentre pondéré. Ce système de calcul comporte :

- un premier sous-système de calcul 146, pour la réalisation du calcul du barycentre brut. Ce premier sous-système comporte les registres 110 et 112 (voir figure 6) pour stocker les valeurs calculées de l'énergie et des coordonnées de la position brute.
- un second sous-système de calcul 148 pour la réalisation du calcul de la pondération. Ce second sous-système est du type décrit ci-dessus en liaison avec la figure 8,
- une troisième sous-système de calcul 150, pour calculer le barycentre pondéré $X_1$, $Y_1$. Ce troisième sous-système a une architecture du type de celle décrite ci-dessus en liaison avec les figures 5 et 6.

La sortie du barycentre pondéré donne les nouvelles coordonnées $X_1$, $Y_1$ de la position de l'événement. De préférence, on prend soin de faire progresser la valeur de l'énergie parallèlement aux calculs ; on obtient en sortie finale, dans un même registre 152, l'énergie et les coordonnées de l'événement.

Le calcul du barycentre pondéré permet d'améliorer la précision de la position.

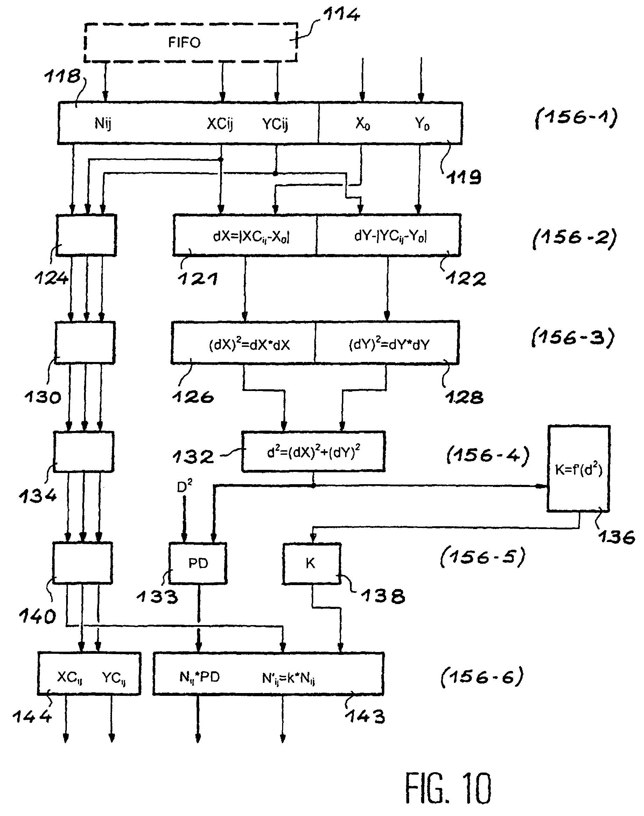

On peut aussi améliorer la précision du calcul sur l'énergie en n'utilisant, pour la calculer, que les photomultiplicateurs qui sont à une distance de $P_0$ inférieure à une valeur déterminée. Ceci permet d'éliminer, parmi les $N_1$ photomultiplicateurs, ceux dont la contribution est constituée essentiellement de bruit. On remarquera que ceux-ci sont d'autant plus nombreux que la position $P_0$ est proche des bords du champ des photomultiplicateurs. Une réalisation de calcul du barycentre pondéré et de l'énergie filtrée est donnée en figure 10.

Sur cette figure, des références identiques à celles de la figure 8 y désignent des éléments identiques ou correspondants.

Les étapes 156-1, 156-2, 156-3, 156-4 sont identiques aux étapes 116-1, 116-21, 116-3, 116-4. De plus, lors de l'étape 156-5, on compare $d^2$ à une valeur $D^2$, où D est la distance, à partir du point d'interaction $P_0$, au-delà de laquelle on estime que le rapport signal/bruit de la contribution d'un photomultiplicateur est trop faible. Le résultat de la comparaison, appelé PD, prend les valeurs suivantes : PD=0 si $d^2>D^2$ et PD=1 si $d^2 \leq D^2$. Ce résultat est stocké dans un registre 133.

Lors de l'étape 156-6, on stocke, dans un registre 143, en plus des valeurs $N'_{i,j}$, $XC_{i,j}$ et $YC_{i,j}$, la valeur $N_{i,j}*PD$ (en fait 0 lorsque $d^2>D^2$ et $N_{i,j}$ lorsque $d^2 \leq D^2$).

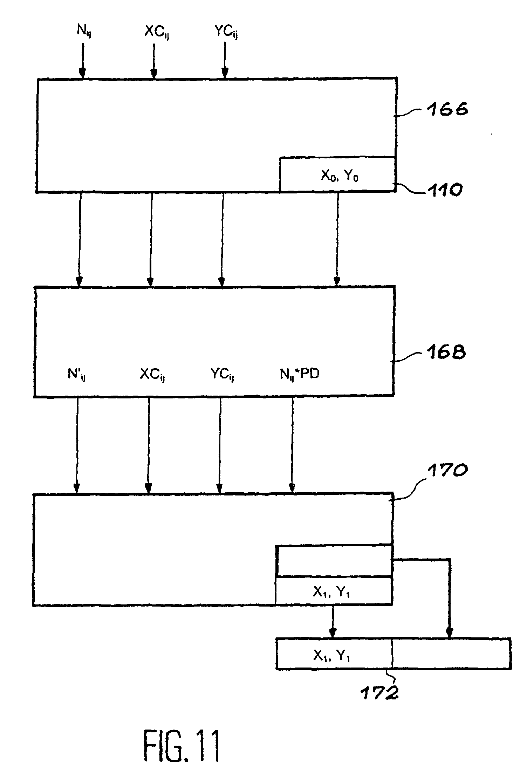

La figure 11 représente schématiquement un système de calcul selon l'invention, pour la mise en oeuvre du calcul du barycentre brut, puis du barycentre pondéré. Ce système de calcul comporte :

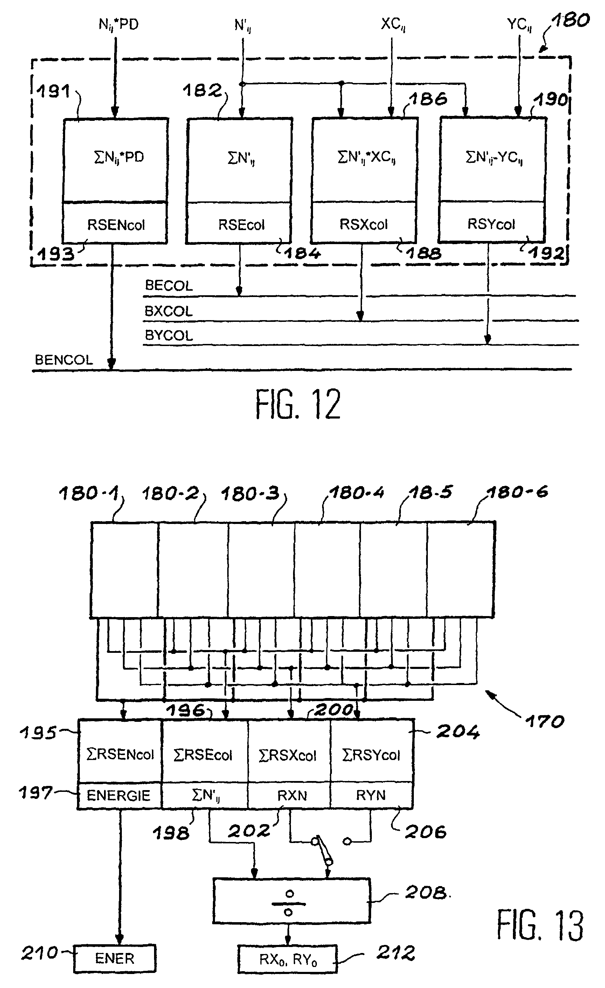

- un premier sous-système de calcul 166, pour la réalisation du calcul du barycentre brut. Ce premier sous-système est semblable au sous-système 146 de la figure 9. Il comporte le registre 110 (voir figure 6) pour stocker les valeurs calculées des coordonnées de la position brute,
- un second sous-système de calcul 168, pour la réalisation du calcul de la pondération. Ce second sous-système est du type décrit ci-dessus en liaison avec la figure 10. Il calcule non seulement $N'_{i,j}$, $XC_{i,j}$, $YC_{i,j}$ mais aussi $N_{i,j}*PD$,
- un troisième sous-système de calcul 170, pour calculer le barycentre pondéré $X_1$, $Y_1$ et la nouvelle valeur de l'énergie. Ce troisième sous-système a une architecture qui va être décrite ci-dessous en liaison avec les figures 12 et 13.

La figure 12 représente un opérateur colonne 180. Celui-ci reçoit, en plus des valeurs $N_{i,j}$ et $N_{i,j}*PD$ des photomultiplicateurs de la colonne, les coordonnées $XC_{i,j}$ et $YC_{i,j}$ des centres des photomultiplicateurs correspondants, fournies par le sous-système de calcul 168.

Chaque opérateur colonne 180 accomplit 4 opérations, de préférence en parallèle.

Une première opération consiste à calculer la contribution de la colonne à l'énergie. Par exemple, après avoir été initialisé en début de séquence, un accumulateur 191 fait la somme des valeurs $N_{i,j}*PD$ des 6 photomultiplicateurs de la colonne et stocke le résultat dans un registre 193 (RSENcol). Les sorties des 6 registres (RSENcol1 à RSENcol6) sont regroupées sur un bus commun BENCOL.

Une deuxième opération consiste à calculer, pour la colonne correspondante la somme $\Sigma N'_{i,j}$. Par exemple, après avoir été initialisé en début de séquence, un accumulateur 182 fait somme $\Sigma N'_{i,j}$ pour les 6 photomultiplicateurs de la colonne, et stocke le résultat dans un registre 184.

Une troisième opération consiste à calculer la contribution de la colonne au barycentre en X. Par exemple, après avoir été initialisé en début de séquence, un multiplieur-accumulateur 186 effectue la somme des contributions au barycentre en X des 6 photomultiplicateurs de la colonne, et stocke le résultat dans un registre 188 (RSXcol). Les sorties des 6 registres (RSXcol1 à RSXco16) sont regroupées sur un bus commun BXCOL.

Une quatrième opération consiste à calculer la contribution de la colonne au barycentre en Y. Par exemple, après avoir été initialisé, un second multiplieur-accumulateur 190 effectue la somme des contributions au barycentre en Y des 6 photomultiplicateurs de la colonne, et stocke le résultat dans un registre 192 (RSYcol). Les sorties des 6 registres (RSYcol1 à RSYcol6) sont regroupées sur un bus commun BYCOL.

A la fin des 6 temps de lecture, les 6 opérateurs colonne ayant effectués leur travail deviennent disponibles pour une nouvelle lecture puisque les résultats de la première lecture sont mémorisés.

Un regroupement des contributions à l'énergie et aux coordonnées du barycentre en X et en Y est ensuite effectué. Ce regroupement va être décrit à l'aide de la figure 13 où les références 180-1, ..., 180-6 désignent 6 opérateurs colonne du type décrit ci-dessus en liaison avec la figure 12.

Un accumulateur 195, après avoir été initialisé en début de séquence, alimenté par le bus BENCOL, effectue la somme des six registres RSENcol1 à RSENcol6 et stocke le résultat dans un registre 197 (ENERGIE). Le contenu de ce registre représente la somme des contributions corrigées $N_{i,j}*PD$ à l'énergie des 36 photomultiplicateurs entourant la position présumée, donc l'énergie de l'événement.

Un second accumulateur 196, après avoir été initialisé en début de séquence, alimenté par le bus BECOL effectue la somme des 6 registres RSEcol1 à RSEcol6 et stocke le résultat dans un registre 198.

Un troisième accumulateur 200, alimenté par le bus BXCOL, effectue la somme des six registres RSXcol1 à RSXcol6, et stocke le résultat dans un registre 202 (RXN). Le contenu de ce registre représente

$$\sum_{i} \left[ \sum_{j} \left( XC_{ij} * N'_{ij} \right) \right].$$

Un quatrième accumulateur 204, alimenté par le bus BYCOL, effectue la somme des six registres RSYcol1 à RSYco16, et stocke le résultat dans un registre 206 (RYN). Le contenu de ce registre représente

$$\sum_{i} \left[ \sum_{j} \left( YC_{ij} * N'_{ij} \right) \right].$$

Les registres RSEcol, RSXcol et RSYcol sont ensuite libérés de façon à pouvoir être utilisés par les opérateurs colonne, et les accumulateurs sont ainsi de nouveau disponibles pour traiter un nouvel événement.

Enfin, les coordonnées $RX_1$ et $RY_1$ du point d'interaction de l'événement sont calculées en effectuant deux divisions à l'aide d'un diviseur 208 :

$$RX_1 = RXN / \Sigma N'_{i,j}$$

$$RY_1 = RYN / \Sigma N'_{i,j}$$

et ceci en moins de 6 temps de lecture pour pouvoir libérer les registres de stockage 198, 202, 206. Les diviseurs intégrés pipelinés du commerce (de type RAYTHEON 3211 par exemple) sont capables d'assumer largement ces performances et permettent même de n'utiliser qu'un seul boîtier, en effectuant les deux divisions successivement.

On obtient donc la position de l'événement dont les coordonnées $RX_1$ et $RY_1$ sont stockées dans un registre 210 ($RX_1$ et $RY_1$). Parallèlement aux divisions, l'énergie est pipelinée depuis le registre 197 vers un registre 212 de façon à libérer le registre 197 pour l'événement suivant.

Les registres 210 et 212 contiennent donc les valeurs des coordonnées corrigées ainsi que la valeur de l'énergie corrigée de l'événement.

Bien que, dans la version générale décrite, le système selon l'invention nécessite une électronique importante, il n'en reste pas moins qu'il est très compétitif par rapport à des systèmes employant des moyens de calcul programmables (microprocesseurs ou DSP). D'autant plus que dans certaines configurations il peut voir sa réalisation notablement simplifiée. C'est le cas par exemple d'une tête de gamma-caméra constituée de photomultiplicateurs carrés dont on suppose que les centres des photomultiplicateurs sont sur une maille régulière et carrée. On peut alors utiliser une notion de pas (distance en X et en Y entre les centres des deux photomultiplicateurs contigus) qui simplifie la plupart des opérateurs.

Il reste à décrire comment le signal issu de chaque photomultiplicateur est détecté et traité, en en particulier comment une position présumée de l'événement peut être calculée.

La figure 14 représente la partie du dispositif associée à un unique photomultiplicateur 60. Le photomultiplicateur 60 relié à un convertisseur courant-tension 262. En réponse à un événement détecté par le photomultiplicateur, on obtient un signal sur la sortie 264 du convertisseur courant-tension 262, par exemple du type de celui qui est illustré sur la figure 15A.

Le graphique de la figure 15A indique, en ordonnées, l'amplitude du signal correspondant à l'impulsion et, en abscisses, le temps. L'amplitude du signal et le temps sont indiqués en échelle arbitraire. $t_0$ désigne l'instant de départ de l'impulsion fournie par le photodétecteur et $t_1$ l'instant où l'impulsion redevient quasiment nulle, après être passée par un maximum. A titre indicatif, la durée correspondant à l'intervalle $t_1 - t_0$ est de l'ordre d'une microseconde, dans le cas d'un photomultiplicateur d'une gamma-caméra couplé à un cristal de NaI(Tℓ).

Le signal analogique présent sur la borne de sortie 264 est dirigé vers un convertisseur analogique-numérique 266. Ce dernier échantillonne chaque impulsion du signal en un certain nombre d'échantillons n, comme illustré sur la figure 15B. Deux échantillons consécutifs sont séparés par un pas, ou intervalle d'horloge p (l'horloge fonctionnant à 1/p Hz).

A titre d'exemple, le convertisseur échantillonne chaque impulsion du signal en n = 10 échantillons. Pour un signal de 1 microseconde, un échantillonnage est alors effectué toutes les 100 nanosecondes.

Le convertisseur analogique-numérique 266 est, de préférence, un convertisseur rapide, de type « flash » pouvant fonctionner à une fréquence de l'ordre de 10 à 20 mégahertz.

Le signal numérique issu du convertisseur analogique-numérique 266 dirigé vers un sommateur numérique 268. Ce sommateur effectue une somme glissante des échantillons qui lui sont transmis par le convertisseur analogique-numérique 266. La somme glissante est effectuée sur un nombre donné d'échantillons. Ce nombre prédéterminé est égal, par exemple, à 10.

Pour chaque photodétecteur i, j, cette somme glissante, ou intégrale numérique du signal délivré en réponse à un événement, correspond à la grandeur $N_{i,j}$ déjà introduite plus haut.

Parallèlement, le résultat de la sommation effectuée avec les moyens 268 est stocké dans un registre 271. La fonction de stockage peut être composée de plusieurs registres pour permettre de mémoriser plusieurs événements temporellement très proches.

La valeur de la somme glissante est dirigée vers des moyens de comparaison 270. La valeur de la somme glissante y est comparée avec une valeur seuil prédéterminée fixée à une entrée 272 du comparateur 270. Ce comparateur émet sur une sortie 274 un signal binaire, représentatif du résultat de la comparaison (par exemple, 0 si la valeur de la somme glissante est inférieure à la valeur de référence fixée et 1 si la valeur de la somme glissante est supérieure à la valeur de référence).

De façon à limiter la durée de ce dépassement, celui-ci ne sera validé que pendant une fenêtre temporelle centrée sur le maximum de la somme glissante. Ceci permet de séparer des événements proches dans le temps mais géographiquement distincts sur le champ du détecteur.

Cette fenêtre est positionnée en prenant comme référence l'instant de passage du signal codé par un maximum. Cette détection est réalisée par les moyens 288 en comparant la valeur courante de la sortie du codeur à la valeur précédente. Lorsque la valeur courante est inférieure à la valeur précédente, le comparateur 288 émet une impulsion. Cette impulsion est envoyée à un registre à décalage 290 dont on règle le retard $n_1$ pour générer une fenêtre temporelle centrée sur le maximum de la somme glissante. Pour tenir compte de l'imprécision (plus ou moins un pas d'échantillonnage) de la détermination de la position du maximum du signal codé, la fenêtre temporelle sera activée pendant $n_0$ pas d'échantillonnage avec $n_0 \geq 3$ (par exemple $n_0 = 3$), ce choix d'un minimum de trois garantissant un minimum de simultanéité des signaux de dépassement de seuil entre les photomultiplicateurs activés par un même événement.

Une porte ET 292, dont les entrées sont le signal obtenu en sortie du comparateur 270, et le signal de sortie du registre à décalage 290, permet d'obtenir, sur sa sortie 294, un signal de dépassement de seuil à l'instant voulu par rapport au passage par le maximum du signal numérique.

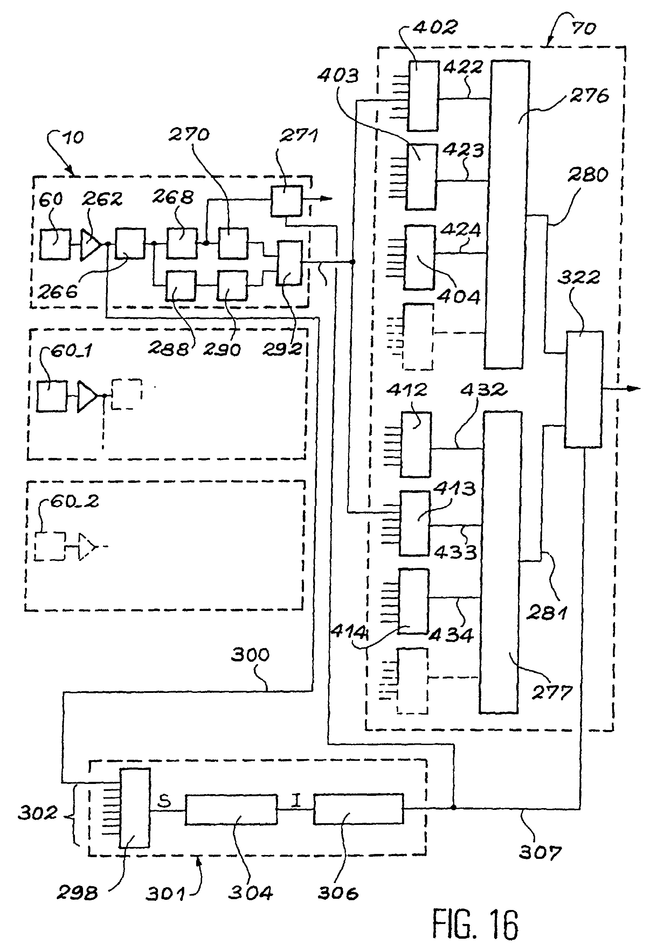

La figure 16 représente un dispositif, conforme à l'invention, pour le traitement des signaux issus de plusieurs photodétecteurs 60, 60-1, 60-2. Sur cette figure, des références identiques à celles de la figure 14 y désignent des éléments similaires ou correspondants.

Sur cette figure, on voit qu'il est possible de prélever, en sortie du convertisseur courant-tension 262, un signal analogique 300, du type de celui qui est décrit ci-dessus en liaison avec la figure 15A. Sur la figure 16, la référence 302 désigne globalement l'ensemble des signaux analogiques prélevés sur les autres convertisseurs courant-tension 262-1, 262-2, .... L'ensemble de ces signaux rentre dans un sommateur analogique 298 qui délivre un signal S, somme de tous les signaux analogiques fournis par un certain nombre de photodétecteurs, par exemple par tous les photodétecteurs. Un dispositif 304 permet de délivrer une impulsion I lors du passage du signal S par son maximum. Ce dispositif 304 comporte, par exemple, un différentiateur (capacité, amplificateur et résistances entre l'entrée et la sortie de l'amplificateur) ; la sortie de ce différentiateur alimente un comparateur qui permet de détecter le passage à 0 de la sortie du différentiateur. L'impulsion I alimente l'entrée d'un registre à décalage 306 dont le pas p est réglé par l'horloge H. La sortie 307 de ce registre est dénommée impulsion de mémorisation et permet, en particulier, de déclencher le registre de mémorisation 271 correspondant au photodétecteur 60. Il déclenche également chaque registre de mémorisation associé à chaque photodétecteur. Le retard du registre à décalage 306 est réglé de façon à ce que le front montant du signal de mémorisation 307 soit synchrone de l'instant où l'on doit mémoriser les sommes dans les registres 271.

L'ensemble des photodétecteurs 60, 60-1, 60-2, ... est réparti par exemple en un réseau bidimensionnel.

Afin de repérer la position présumée (ou grossière) d'un événement par rapport à ce réseau bidimensionnel de photodétecteurs, on associe avantageusement une mémoire à accès lecture à une première direction de repérage dans le réseau de photodétecteurs et une mémoire à accès lecture à une seconde direction de repérage dans le réseau des photodétecteurs. Si on repère ce réseau par des lignes et des colonnes, on peut donc ainsi associer une mémoire à accès lecture pour repérer une coordonnée « ligne » et une mémoire à accès lecture pour repérer une coordonnée « colonne ».

Plus précisément, dans un dispositif selon l'invention, du type de celui illustré en figure 6, les sorties 294 qui représentent, lorsqu'elles sont actives, les photodétecteurs du centre de l'interaction, sont utilisées de la manière suivante :

- un circuit OU (402) regroupe les sorties de type 294 des photodétecteurs d'une même colonne et génère un signal 422 actif lorsqu'au moins une de ses entrées est active. Il y a autant de circuits de type 402 que de colonnes,
- un circuit OU (412) regroupe les sorties de type 294 des photodétecteurs d'une même ligne et génère un signal 432 actif lorsqu'au moins une de ses entrées est active. Il y a autant de circuits de type 412 que de lignes.

Les signaux de type 422 sont les adresses d'une PROM 276 qui. est programmée de façon à fournir la coordonnée (280) de la position présumée par rapport aux colonnes. De même, les signaux de type 432 sont les adresses d'une deuxième PROM 277 qui est programmée de façon à fournir la coordonnée (281) de la position présumée par rapport aux lignes. La position présumée, représentée par le couple de valeurs (280, 281), est mémorisée dans un registre 322, en même temps que l'on mémorise la contribution de tous les photodétecteurs dans leurs registres respectifs (271). Cette mémorisation est déclenchée par le signal 307 généré par le registre 306.

**Revendications**

**1.** Procédé de détermination de la position $P_0$ d'un événement par rapport à un ensemble de N photodétecteurs, cet événement induisant un signal dans les N photodétecteurs, ce procédé comportant les étapes suivantes :

    a) une étape de numérisation du signal délivré par chaque photodétecteur, et de calcul d'une valeur $N_{i,j}$ représentative de l'énergie du signal délivré par chaque photodétecteur,
    procédé **caractérisé en ce qu'**il comporte en outre :
    b) une étape de détermination, pour chaque colonne i :

    - de la contribution de la colonne à l'énergie totale induite par l'événement dans l'ensemble des photodétecteurs,
    - de la contribution de la colonne au barycentre, en X, de l'événement,
    - de la contribution de la colonne au barycentre, en Y, de l'événement,

    c) une étape de détermination :

- de l'énergie totale induite par l'événement dans l'ensemble des photodétecteurs, par sommation des contributions de chaque colonne à l'énergie totale calculées à l'étape b),
- des coordonnées barycentriques $(X_0, Y_0)$ de l'événement par rapport aux N photodétecteurs par la méthode du barycentre.

2. Procédé selon la revendication 1, comportant une étape préliminaire de détection de la position présumée d'un événement.

3. Procédé selon la revendication 2, comportant en outre une étape de délimitation d'un sous-ensemble de $N_1$ photodétecteurs parmi l'ensemble de N photodétecteurs, autour de la position présumée de l'événement, seuls les signaux des $N_1$ photodétecteurs de ce sous-ensemble étant traités selon les étapes b) et c).

4. Procédé selon l'une des revendications 1 à 3, comportant en outre, après la détermination des coordonnées barycentriques $(X_0,Y_0)$ de l'événement par rapport aux N photodétecteurs, les étapes suivantes :

   d) détermination de la distance $d_{i,j}$ entre $P_0$ et chacun des photodétecteurs dont les signaux interviennent dans les étapes b) et c),
   e) pondération du signal $N_{i,j}$ délivré par chacun des photodétecteurs, dont les signaux interviennent dans les étapes b) et c), pour obtenir une valeur de signal pondéré $N'_{i,j}=K*N_{i,j}$, où K est une fonction de $d_{i,j}$.

5. Procédé selon la revendication 4, la fonction K étant :

   - inférieure à 1 pour $d_{i,j}$ inférieure à la dimension du photodétecteur,
   - supérieure à 1 pour $d_{i,j}$ supérieure ou de l'ordre de grandeur de la dimension du photodétecteur,
   - sensiblement égale à 0 pour $d_{i,j}$ grand devant la dimension du photodétecteur.

6. Procédé selon la revendication 4 ou 5, comportant en outre :

   b') une étape de détermination, à l'aide des valeurs de signal pondéré $N'_{i,j}$, pour chaque colonne i :

   - de la contribution de la colonne à l'énergie totale induite par l'événement dans l'ensemble des photodétecteurs,
   - de la contribution de la colonne au barycentre, en X, de l'événement,
   - de la contribution de la colonne au barycentre, en Y, de l'événement.

   c') Une étape de détermination, à l'aide des valeurs de signal pondéré $N'_{i,j}$ :

   - de l'énergie totale induite par l'événement dans l'ensemble des photodétecteurs,
   - des nouvelles coordonnées barycentriques $(X'_0, Y'_0)$ de l'événement par rapport aux N photodétecteurs.

7. Procédé selon l'une des revendications 1 à 6, comportant en outre, après la détermination des coordonnées barycentriques $P_0(X_0,Y_0)$ de l'événement par rapport aux N photodétecteurs, les étapes suivantes :

   d') détermination de la distance $d_{i,j}$ entre $P_0$ et chacun des photodétecteurs, dont les signaux interviennent dans les étapes b) et c),
   f) pour chaque $d_{i,j}$, comparaison avec une valeur D,
   g) détermination, pour chaque photodétecteur, de la valeur $N_{i,j}*PD$, où PD=0 si $d_{i,j}>D$ et PD≠0 si $d_{i,j}≤D$.

8. Procédé selon la revendication 7, comportant en outre une étape

   h) de détermination d'une nouvelle valeur de l'énergie totale en fonction des valeurs $N_{i,j}*PD$.

9. Procédé selon la revendication 8, la détermination d'une nouvelle valeur de l'énergie selon h) comportant :

   b") une étape de détermination, pour chaque colonne, en fonction des valeurs $N_{i,j}*PD$, de la contribution de la colonne à l'énergie totale induite par l'événement dans l'ensemble des photodétecteurs,
   c") une étape de détermination de l'énergie totale induite par l'événement dans l'ensemble des photodétecteurs, en faisant la somme des contributions obtenues en b") pour les différentes colonnes.

**10.** Procédé selon l'une des revendications 1 à 9, les photodétecteurs étant des photomultiplicateurs d'une gamma-caméra.

**11.** Procédé d'imagerie en correction d'atténuation par transmission, mettant en oeuvre le procédé selon la revendication 10.

**12.** Procédé d'imagerie en PET en coïncidence mettant en oeuvre le procédé selon la revendication 10.

**13.** Dispositif pour la détermination de la position $P_0$ d'un événement par rapport à un ensemble de N photodétecteurs, cet événement induisant un signal dans les N photodétecteurs, ce dispositif comportant :

a) des moyens (266, 268) pour numériser un signal délivré par chaque photodétecteur (60), et pour calculer une valeur $N_{i,j}$ représentative de l'énergie du signal délivré par chaque photodétecteur,
dispositif **caractérisé en ce qu'**il comporte en outre :
b) des moyens (82, 86, 90) pour déterminer, pour chaque colonne i :

- la contribution de la colonne à l'énergie totale induite par l'événement dans l'ensemble des photodétecteurs,
- la contribution de la colonne au barycentre, en X, de l'événement,
- la contribution de la colonne au barycentre, en Y, de l'événement.

c) des moyens (96, 100, 104, 108) pour déterminer :

- par sommation des contributions de chaque colonne, l'énergie totale induite par l'événement dans l'ensemble des photodétecteurs,
- les coordonnées barycentriques $(X_0, Y_0)$ de l'événement par rapport aux N photodétecteurs.

**14.** Dispositif selon la revendication 13, comportant des moyens (70) pour détecter la position présumée d'un événement.

**15.** Dispositif selon la revendication 14, comportant en outre des moyens (68, 74) pour délimiter un sous-ensemble de $N_1$ photodétecteurs parmi l'ensemble de N photodétecteurs, autour de la position présumée de l'événement.

**16.** Dispositif selon l'une des revendications 13 à 15, comportant en outre des moyens (148) pour :

d) déterminer la distance $d_{i,j}$ entre $P_0$ et chacun des photodétecteurs dont les signaux interviennent dans les calculs de l'énergie du barycentre
e) pondérer le signal $N_{i,j}$ délivré par chacun des photodétecteurs, dont les signaux interviennent dans les calculs de l'énergie et du barycentre, pour obtenir une valeur de signal pondéré $N'_{i,j}=K*N_{i,j}$, où K est une fonction de $d_{i,j}$.

**17.** Dispositif selon la revendication 16, la fonction K étant :

- inférieure à 1 pour $d_{i,j}$ inférieure à la dimension d'un photodétecteur,
- supérieure à 1 pour $d_{i,j}$ supérieure ou de l'ordre de grandeur de la dimension d'un photodétecteur,
- sensiblement égale à 0 pour $d_{i,j}$ grand devant la dimension du photodétecteur.

**18.** Dispositif selon l'une des revendications 16 ou 17, comportant en outre des moyens (150) pour :

b') déterminer, à l'aide des valeurs de signal pondéré $N'_{i,j}$, pour chaque colonne i :

- la contribution de la colonne à l'énergie totale induite par l'événement dans l'ensemble des photodétecteurs,
- la contribution de la colonne au barycentre, en X, de l'événement,
- la contribution de la colonne au barycentre, en Y, de l'événement.

c') déterminer, à l'aide des valeurs des contributions obtenues en b') :

- l'énergie totale induite par l'événement dans l'ensemble des photodétecteurs,
- des nouvelles coordonnées barycentriques $(X_1, Y_1)$ de l'événement par rapport aux N photodétecteurs.

**19.** Dispositif selon l'une des revendications 13 à 18, comportant en outre des moyens pour :

d') déterminer la distance $d_{i,j}$ entre $P_0$ et chacun des photodétecteurs, dont les signaux interviennent dans les calculs du barycentre et de l'énergie,
f) comparer chaque $d_{i,j}$, avec une valeur D,
g) déterminer, pour chaque photodétecteur dont les signaux interviennent dans les calculs du barycentre et de l'énergie, la valeur $N_{i,j}*PD$, où PD=0 si $d_{i,j}$>D et PD≠0 si $d_{i,j}$≤D.

**20.** Dispositif selon la revendication 19, comportant en outre des moyens pour déterminer une nouvelle valeur de l'énergie totale en fonction des valeurs $N_{i,j}*PD$.

**21.** Dispositif selon la revendication 20, les moyens pour déterminer une nouvelle valeur de l'énergie comportant :

- des moyens (191) de détermination, pour chaque colonne, en fonction des valeurs $N_{i,j}*PD$, de la contribution de la colonne à l'énergie totale induite par l'événement dans l'ensemble des photodétecteurs,
- des moyens (195) de détermination de l'énergie totale induite par l'événement dans l'ensemble des photodétecteurs.

**22.** Dispositif selon l'une des revendications 13 à 21, les photodétecteurs étant des photomultiplicateurs d'une gamma-caméra.

**Claims**

**1.** Process for determining the position Po of an event with respect to a set of N photodetectors, this event inducing a signal in the N photodetectors, this process comprising the following steps:

a) a step in which the signal delivered by each photodetector is digitized, and a value $N_{i,j}$ representing the energy of the signal delivered by each photodetector is calculated,
process **characterized in that** it also comprises:
b) a step in which the following are determined for each column i:

- the contribution of the column to the total energy induced by the event in the set of photodetectors,
- the contribution of the column to the X value of the centre of gravity of the event,
- the contribution of the column to the Y value of the centre of gravity of the event,

c) a step in which the following are determined:

- the total energy induced by the event in the set of photodetectors,
- the coordinates of the centre of gravity (Xo, Yo) of the event with respect to the N photodetectors.

**2.** Process according to claim 1 comprising a preliminary step for detection of the presumed position of an event.

**3.** Process according to claim 2, also comprising a step for the delimitation of a sub-set of $N_1$ photodetectors among the set of N photodetectors around the presumed position of the event, only the signals from the $N_1$ photodetectors of this sub-set being processed according to steps b) and c).

**4.** Process according to one of claims 1 to 3, also comprising the following steps after the coordinates of the centre of gravity (Xo, Yo) of the event have been determined with respect to the N photodetectors:

d) determine the distance $d_{i,j}$ between Po and each of the photodetectors for which the signals are used in steps b) and c),
e) weight the signal $N_{i,j}$ output by each photodetector, for which the signals are used in steps b) and c) to obtain a weighted signal value $N'_{i,j} = K*N_{i,j}$ where K is a function of $d_{i,j}$.

**5.** Process according to claim 4, the function K being:

- less than 1 if $d_{i,j}$ is greater than or of the same order of magnitude as the size of the photodetector,
- approximately equal to 0 if $d_{i,j}$ is large compared with the size of the photodetector.

**6.** Process according to one of claims 4 or 5, also comprising:

b') a step in which the following are determined using the values of the weighted signal, $N'_{i,j}$ for each column i:

- the contribution of the column to the total energy induced by the event in the set of photodetectors,
- the contribution of the column to the X value of the centre of gravity of the event,
- the contribution of the column to the Y value of the centre of gravity of the event;

c') a step in which the following are determined using the values of the weighted signal, $N'_{i,j}$:

- the total energy induced by the event in the set of photodetectors,
- the new coordinates of the centre of gravity (X'o, Y'o) of the event with respect to the N photodetectors.

**7.** Process according to one of claims 1 to 6, also comprising the following steps after the coordinates of the centre of gravity Po (Xo, Yo) of the event with respect to the N photodetectors have been determined:

d') determine the distance $d_{i,j}$ between Po and each photodetector the signals of which are used in steps b) and c),
f) compare with a value D, for each value of $d_{i,j}$,
g) determine the value $N_{i,j}*PD$, where PD = 0 if $d_{i,j} > D$ and PD $\neq$ 0 if $d_{i,j} \leq D$ for each photodetector.

**8.** Process according to claim 7, also comprising a step

h) to determine a new value of the total energy as a function of the values $N_{i,j}*PD$.

**9.** Process according to claim 8, the new value of the energy according to step h) being determined in the following steps:

b") determine the contribution of each column to the total energy induced by the event in the set of photodetectors, as a function of the values $N_{i,j}*PD$,
c") determine the total energy induced by the event in the set of photodetectors, by taking the sum of the contributions obtained in b") for the various columns.

**10.** Process according to one of claims 1 to 9, the photodetectors being photo-multipliers of a gamma-camera.

**11.** Imagery process in correction of transmission attenuation, embodying the process according to claim 10.

**12.** PET coincidence imagery process embodying the process according to claim 10.

**13.** Device for determining the position Po of an event with respect to a set of N photodetectors, this event inducing a signal in the N photodetectors, this device comprising:

a) means (266, 268) of digitizing signals delivered by each photodetector (60), and of calculating a value $N_{i,j}$ representing the energy of the signal delivered by each photodetector,
device **characterized in that** it also comprises:
b) means (82, 86, 90) of determining, for each column i:

- the contribution of the column to the total energy induced by the event in the set of photodetectors,
- the contribution of the column to the x value of the centre of gravity of the event,
- the contribution of the column to the Y value of the centre of gravity of the event,

c) means (96, 100, 104, 108) of determining:

- the total energy induced by the event in the set of photodetectors,
- the coordinates of the centre of gravity (Xo, Yo) of the event with respect to the N photodetectors.

**14.** Device according to claim 13, also comprising means (70) of detecting the presumed position of an event.

**15.** Device according to claim 14, also comprising means (68, 74) of delimiting a subset of $N_1$ photodetectors among the set of N photodetectors around the presumed position of the event.

**16.** Device according to one of claims 13 to 15, also comprising means (148) for:

d) determining the distance $d_{i,j}$ between Po and each photodetector for which the signals are used to calculate the energy and the centre of gravity,
e) weighting the signal $N_{i,j}$ output by each photodetector for which the signals are used to calculate the energy and the centre of gravity, to obtain a weighted signal value $N_{i,j} = K*N_{i,j}$ where K is a function of $d_{i,j}$.

**17.** Device according to claim 16, in which the function K is

- less than 1 if $d_{i,j}$ is less than the size of the photodetector,
- greater than 1 if $d_{i,j}$ is greater than or of the same order of magnitude as the size of the photodetector,
- approximately equal to 0 if $d_{i,j}$ is large compared with the size of the photodetector.

**18.** Device according to one of claims 16 or 17, also comprising means (150) of:

b') determining the following for each column i making use of the values of the weighted signal $N'_{i,j}$:

- the contribution of the column to the total energy induced by the event in the set of photodetectors,
- the contribution of the column to the X value of the centre of gravity of the event,
- the contribution of the column to the Y value of the centre of gravity of the event,

c') determining the following making use of the values of the contributions obtained in b'):

- the total energy induced by the event in the set of photodetectors,
- the new coordinates of the centre of gravity $(X_1, Y_1)$ of the event with respect to the N photodetectors.

**19.** Device according to one of claims 13 to 18, also comprising means of:

d') determining the distance $d_{i,j}$ between $P_0$ and each photodetector, for which the signals are used to calculate the centre of gravity and the energy,
f) comparing each value of $d_{i,j}$ with a value D,
g) determining the value $N_{i,j}*PD$, where PD = 0 if $d_{i,j} > D$ and PD = 0 if $d_{i,j} \leq D$, for each photodetector for which the signals are used to calculate the centre of gravity and the energy.

**20.** Device according to claim 19, also comprising means of determining a new value of the total energy as a function of the values $N_{i,j}*PD$.

**21.** Device according to claim 20, the means of determining a new value of the energy comprising:

b") means (191) of determining the contribution of each column to the total energy induced by the event in the set of photodetectors, as a function of the values $N_{i,j}*PD$,
c") means (195) of determining the total energy induced by the event in the set of photodetectors.

**22.** Device according to one of claims 13 to 21, the photodetectors being the photo-multipliers in a gamma-camera.

**Patentansprüche**

**1.** Verfahren zur Bestimmung der Position $P_0$ eines Ereignisses in Bezug auf eine Anordnung bzw. Menge von N Photodetektoren, wobei dieses Ereignis in den N Photodetektoren ein Signal induziert und dieses Verfahren folgenden Schritt umfasst:

a) einen Schritt des Digitalisierens des von jedem Photodetektor gelieferten Signals und des Berechnens eines Werts $N_{i,j}$, repräsentativ für die Energie des von jedem Photodetektor gelieferten Signals, wobei dieses Verfahren **dadurch gekennzeichnet ist, dass** es außerdem folgende Schritte umfasst:
b) einen Schritt zur Feststellung für jede Spalte i:

- des Beitrags der Spalte zu der durch das Ereignis in der Gesamtheit der Photodetektoren induzierten Gesamtenergie,
- der Beitrag der Spalte zum Baryzentrum bzw. Schwerpunkt des Ereignisses in X,
- der Beitrag der Spalte zum Baryzentrum bzw. Schwerpunkt des Ereignisses in Y,

c) einen Schritt zur Ermittlung:

- der durch das Ereignis in der Gesamtheit der Photodetektoren induzierten Gesamtenergie durch Summierung der Beiträge von jeder Spalte zu der in Schritt b) berechneten Gesamtenergie,
- Baryzentrums- bzw. Schwerpunktkoordinaten $(X_0, Y_0)$ des Ereignisses in Bezug auf die N Photodetektoren durch die Schwerpunktmethode.

2. Verfahren nach Anspruch 1 mit einem vorbereitenden Schritt zu Detektion der angenommenen Position eines Ereignisses.

3. Verfahren nach Anspruch 2 mit außerdem einem Schritt zur Abgrenzung einer Teilmenge von $N_1$ Photodetektoren aus der Gesamtheit der N Photodetektoren, um die angenommene Position des Ereignisses herum, wobei nur die Signale der $N_1$ Photodetektoren dieser Teilmenge gemäß den Schritten b) und c) verarbeitet werden.

4. Verfahren nach einem der Ansprüche 1 bis 3 mit außerdem - nach der Ermittlung der Schwerpunktskoordinaten $(X_0, Y_0)$ des Ereignisses in Bezug auf die N Photodetektoren - den folgenden Schritten:

d) Feststellung des Abstands $d_{i,j}$ zwischen $P_0$ und jedem der Photodetektoren, dessen Signale sich in den Schritten b) und c) ereignen,
e) Gewichtung des Signals $N_{i,j}$, geliefert von jedem der Photodetektoren, dessen Signale sich in den Schritten b) und c) ereignen, um einen Wert eines gewichteten Signals $N'_{i,j}=K*N_{i,j}$ zu erhalten, wo K eine Funktion von $d_{i,j}$ ist.

5. Verfahren nach Anspruch 4, wobei die Funktion K:

- kleiner ist als 1 für $d_{i,j}$ kleiner als die Dimension des Photodetektors,
- größer ist als 1 für $d_{i,j}$ größer als die Dimension des Photodetektors oder gleich groß,
- im Wesentlichen gleich 0 ist für $d_{i,j}$ groß gegenüber der Dimension des Photodetektors.

6. Verfahren nach Anspruch 4 oder 5, außerdem umfassend:

b') einen Schritt, um mit Hilfe der Werte des gewichteten Signals $N'_{i,j}$ für jede Spalte i festzustellen:

- den Beitrag der Spalte zu der durch das Ereignis in der Gesamtheit der Photodetektoren induzierten Gesamtenergie,
- den Beitrag der Spalte zum Baryzentrum bzw. Schwerpunkt des Ereignisses in X,
- den Beitrag der Spalte zum Baryzentrum bzw. Schwerpunkt des Ereignisses in Y,

c') einen Schritt, um mit Hilfe der Werte des gewichteten Signals $N'_{i,j}$ festzustellen:

- die durch das Ereignis in der Gesamtheit der Photodetektoren induzierte Gesamtenergie,
- neue Baryzentrums- bzw. Schwerpunktkoordinaten $(X'_0, Y'_0)$ des Ereignisses in Bezug auf die N Photodetektoren.

7. Verfahren nach einem der Ansprüche 1 bis 6, außerdem - nach Ermittlung der Schwerpunktkoordinaten $P_0(X_0, Y_0)$ des Ereignisse in Bezug auf die N Photodetektoren -, die folgenden Schritte umfassend:

d') Ermittlung des Abstands $d_{i,j}$ zwischen $P_0$ und jedem der Photodetektoren, dessen Signale sich in den Schritten b) und c) ereignen,
f) Vergleich jedes $d_{i,j}$ mit einem Wert D,
g) Ermittlung - für jeden Photodetektor - des Werts $N_{i,j}$*PD, wo PD=0 wenn $d_{i,j}$>D, und PD≠0 wenn $d_{i,j}$≤D.

8. Verfahren nach Anspruch 7, außerdem umfassend einen Schritt

   h) zur Ermittlung eines neuen Werts der Gesamtenergie in Abhängigkeit von den Werten $N_{i,j}$*PD.

9. Verfahren nach Anspruch 8, bei dem die Ermittlung eines neuen Werts der Energie gemäß h) umfasst:

   b") einen Schritt zur Feststellung - für jede Spalte in Abhängigkeit der Werte $N_{i,j}$*PD - des Beitrags der Spalte zu der durch das Ereignis in der Gesamtheit der Photodetektoren induzierten Gesamtenergie,
   c") einen Schritt zur Feststellung der durch das Ereignis in der Gesamtheit der Photodetektoren induzierten Gesamtenergie, indem man die in b") für die verschiedenen Spalten erhaltenen Beiträge summiert.

10. Verfahren nach einem der Ansprüche 1 bis 9, bei dem die Photodetektoren Photovervielfacher einer Gamma-Kamera sind.

11. Verfahren zur Transmissionsdämpfungskorrektur-Bildherstetlung, bei dem das Verfahren nach Anspruch 10 angewendet wird.

12. Verfahren zur Koinzidenz-PET-Bildherstellung, bei dem das Verfahren nach Anspruch 10 angewendet wird.

13. Vorrichtung zur Feststellung der Position $P_0$ eines Ereignisses in Bezug auf eine Anordnung bzw. Menge von N Photodetektoren, wobei dieses Ereignis in den N Photodetektoren ein Signal induziert und diese Vorrichtung dabei umfasst:

    a) Einrichtungen (266, 268) zum Digitalisieren des von jedem Photodetektor gelieferten Signals und des Berechnens eines Werts $N_{i,j}$, repräsentativ für die Energie des von jedem Photodetektor gelieferten Signals, wobei dieses Vorrichtung **dadurch gekennzeichnet ist, dass** sie außerdem umfasst:
    b) Einrichtungen (82, 86, 90) zur Feststellung für jede Spalte i:

    - des Beitrags der Spalte zu der durch das Ereignis in der Gesamtheit der Photodetektoren induzierten Gesamtenergie,
    - der Beitrag der Spalte zum Baryzentrum bzw. Schwerpunkt des Ereignisses in X,
    - der Beitrag der Spalte zum Baryzentrum bzw. Schwerpunkt des Ereignisses in Y,

    c) Einrichtungen (96, 100, 104, 108) zur Ermittlung:

    - der durch das Ereignis in der Gesamtheit der Photodetektoren induzierten Gesamtenergie durch Summierung der Beiträge von jeder Spalte zu der in Schritt b) berechneten Gesamtenergie,
    - Baryzentrums- bzw. Schwerpunktkoordinaten ($X_0$, $Y_0$) des Ereignisses in Bezug auf die N Photodetektoren durch die Schwerpunktmethode.

14. Vorrichtung nach Anspruch 13, Einrichtungen (70) zum Detektieren der angenommenen Position eines Ereignisses umfassend.

15. Vorrichtung nach Anspruch 14 mit außerdem Einrichtungen (68, 74) zur Abgrenzung einer Teilmenge von $N_1$ Photodetektoren aus der Gesamtheit der N Photodetektoren, um die angenommene Position des Ereignisses herum.

16. Vorrichtung nach einem der Ansprüche 13 bis 15 mit außerdem Einrichtungen (148);

    d) zur Feststellung des Abstand $d_{i,j}$ zwischen $P_0$ und jedem der Photodetektoren, dessen Signale bei den Berechnungen der Energie des Baryzentrums bzw. Schwerpunkts beteiligt sind,
    e) zur Gewichtung des Signals $N_{i,j}$, geliefert von jedem der Photodetektoren, dessen Signale bei den Berechnungen der Energie des Baryzentrums bzw. Schwerpunkts beteiligt sind, um einen Wert eines gewichteten Signals $N'_{i,j}$=K*$N_{i,j}$ zu erhalten, wo K eine Funktion von $d_{i,j}$ ist.

**17.** Vorrichtung nach Anspruch 16, wobei die Funktion K:

- kleiner ist als 1 für $d_{i,j}$ kleiner als die Dimension eines Photodetektors,
- größer ist als 1 für $d_{i,j}$ größer als die Dimension des Photodetektors oder gleich groß,
- im Wesentlichen gleich 0 ist für $d_{i,j}$, das groß ist gegenüber der Dimension des Photodetektors.

**18.** Vorrichtung nach einem der Ansprüche 16 oder 17, außerdem Einrichtungen (150) umfassend:

b') um mit Hilfe der Werte des gewichteten Signals $N'_{i,j}$ für jede Spalte i festzustellen:

- den Beitrag der Spalte zu der durch das Ereignis in der Gesamtheit der Photodetektoren induzierten Gesamtenergie,
- den Beitrag der Spalte zum Baryzentrum bzw. Schwerpunkt des Ereignisses in X,
- den Beitrag der Spalte zum Baryzentrum bzw. Schwerpunkt des Ereignisses in Y,

c') um mit Hilfe der in b') erhaltenen Werte des gewichteten Signals $N'_{i,j}$ festzustellen:

- die durch das Ereignis in der Gesamtheit der Photodetektoren induzierte Gesamtenergie,
- neue Baryzentrums- bzw. Schwerpunktkoordinaten $(X_1, Y_1)$ des Ereignisses in Bezug auf die N Photodetektoren.

**19.** Vorrichtung nach einem der Ansprüche 13 bis 18 mit außerdem Einrichtungen:

d') zum Ermitteln des Abstands $d_{i,j}$ zwischen $P_0$ und jedem der Photodetektoren, dessen Signale sich in den Schritten b) und c) ereignen,
f) zum Vergleichen jedes $d_{i,j}$ mit einem Wert D,
g) zum Ermitteln - für jeden Photodetektor, dessen Signale an den Berechnungen des Schwerpunkts und der Energie beteiligt sind - des Werts $N_{i,j}$*PD, wo PD=0 wenn $d_{i,j}$>D, und PD≠0 wenn $d_{i,j}$≤D.

**20.** Vorrichtung nach Anspruch 19 mit außerdem Einrichtungen zum Ermitteln eines neuen Werts der Gesamtenergie in Abhängigkeit von den Werten $N_{i,j}$*PD.

**21.** Vorrichtung nach Anspruch 20, bei der die Einrichtungen Ermittlung eines neuen Werts der Energie umfassen:

- Einrichtungen (191) zur Feststellung - für jede Spalte in Abhängigkeit den Werten $N_{i,j}$*PD - des Beitrags der Spalte zu der durch das Ereignis in der Gesamtheit der Photodetektoren induzierten Gesamtenergie,
- Einrichtungen (195) zur Feststellung der durch das Ereignis in der Gesamtheit der Photodetektoren induzierten Gesamtenergie.

**22.** Vorrichtung nach einem der Ansprüche 13 bis 21, bei der die Photodetektoren Photovervielfacher einer Gamma-Kamera sind.

FIG. 1

FIG. 2B

FIG. 2A

LX_

LX+

52X_     54X_

52X+     54X+

57     58

52Y_     54Y_

LY_

56

LY+

52Y+     54Y+

26

Rx_     Ry+

50

Ry_     Rx+

EP 0 950 196 B1

FIG. 3

FIG. 4

$N_{ij}$        $XC_{ij}$        $YC_{ij}$        $80$

$94$   $82$   $\sum N_{ij}$   $86$   $\sum N_{ij} \cdot XC_{ij}$   $\sum N_{ij} \cdot YC_{ij}$   $90$

RSEcol   RSXcol   RSYcol

$84$   $88$   $92$

BECOL

BXCOL

BYCOL

# FIG. 5

$80 \cdot 1$   $80 \cdot 2$   $80 \cdot 3$   $80 \cdot 4$   $80 \cdot 5$   $80 \cdot 6$

$76$   $96$   $100$   $104$

$\sum$RSEcol   $\sum$RSXcol   $\sum$RSYcol

ENERGIE   RXN   RYN

$98$   $102$   $106$

# FIG. 6

$108$

$112$   ENER   RX$_0$, RY$_0$   $110$

FIG. 7A

FIG. 7B

FIG. 8

$N_{ij}$    $XC_{ij}$    $YC_{ij}$

146

112

110

$N'_{ij}$    $XC_{ij}$    $YC_{ij}$

148

150

$X_1, Y_1$

$X_1, Y_1$

152

FIG. 9

FIG. 10

FIG. 11

FIG. 12

FIG. 13

FIG. 14

FIG. 15 A

FIG. 15 B

FIG. 16